# EUROPEAN PATENT APPLICATION

(11) **EP 3 904 513 A1**
(43) Date of publication of application: **03.11.2021**
(21) Application number: 20172036.4
(22) Date of filing: 29.04.2020
(51) Int. Cl.: C12N 15/10, G01N 33/68

(54) **UNBLENDING OF TRANSCRIPTIONAL CONDENSATES IN HUMAN REPEAT EXPANSION DISEASE**

(71) Applicant: Max-Planck-Gesellschaft zur Förderung der Wissenschaften e.V., 80539 München (DE)
(72) Inventor: The designation of the inventor has not yet been filed
(74) Representative: Weiss, Wolfgang

(57) **Abstract**

The present invention relates to a method of determining the capacity of a Cluster 1 mammalian Transcription Factor for phase separation and/or the capacity for forming a transcriptional condensate in a sample. Further, the present invention relates to an active agent for use in a method of preventing and/or treating a disorder associated with, caused by and/or accompanied with a dysfunction of a biomolecular condensate comprising at least one Cluster 1 mammalian Transcription Factor.

SEQUENCE LISTING

<110>  MPG Max-Planck-Gesellschaft zur Förderung der
       Wissenschaften e. V.

<120>  Unblending of transcriptional condensates

<130>  70644P EP

<140>  N/A
<141>  2020-04-29

<160>  42

<170>  PatentIn version 3.5

<210>  1
<211>  94
<212>  DNA
<213>  artificial sequence

<220>
<223>  SV40 NLS forward primer


<220>
<221>  misc_binding
<222>  (1)..(1)
<223>  5Phos

<400>  1
ggccggaact cccacctgca acatgcgtga cggaggcggt ccaaaaaaga agagaaaggt      60

atgactgagg ccgcgactct agagtcgacc tgca                                  94


<210>  2
<211>  86
<212>  DNA
<213>  artificial sequence

<220>
<223>  SV40 NLS reverse primer


<220>
<221>  misc_binding
<222>  (1)..(1)
<223>  5Phos

<400>  2
ggtcgactct agagtcgcgg cctcagtcat acctttctct tcttttttgg accgcctccg      60

tcacgcatgt tgcaggtggg agttcc                                           86


<210>  3
<211>  83
<212>  DNA
<213>  artificial sequence

<220>
<223>  +7A oligonucleotide for Gibson assembly

<400>  3
gcggctgctg ctgctgctgc agccgcggcg gcagctgcag ctgcggccgc tgccgctagt      60

ggatttgcct atcctgggac gag                                              83


<210>  4
<211>  86
<212>  DNA
<213>  artificial sequence

<220>
<223>  +8A oligonucleotide for Gibson assembly

<400>  4
gcggctgctg ctgctgctgc agccgcggcg gcagctgcag ctgcggccgc agctgccgct      60

agtggatttg cctatcctgg gacgag                                           86


<210>  5
<211>  89
<212>  DNA
<213>  artificial sequence

<220>
<223>  +9A oligonucleotide for Gibson assembly

<400>  5
gcggctgctg ctgctgctgc agccgcggcg gcagctgcag ctgcggccgc agcagctgcc      60

gctagtggat ttgcctatcc tgggacgag                                        89


<210>  6
<211>  104
<212>  DNA
<213>  artificial sequence

<220>
<223>  +14A oligonucleotide for Gibson assembly

<400>  6
gcggctgctg ctgctgctgc agccgcggcg gcagcagctg cagcggctgc tgcagctgcg      60

gccgcagcag ctgccgctag tggatttgcc tatcctggga cgag                      104


<210>  7
<211>  507
<212>  DNA
<213>  artificial sequence

<220>
<223>  Hoxd13 wt gene fragment

<400>  7
cgagctctat aaaagagctc acaacccctc actcggcgcg ccagtcctcc gacagactga      60

gtcgcccggg ggggatctgg agctctcgag aattctcacg cgtcaagtgg agcaaggcag     120

gtggacagtg gatccggagc taccatgttt gcgggcacac attccgggag ggcggcggca     180

gctgctgcag ctgctgcggc agctgctgcc gcggcagcgg cagctgctgc agctgctagt     240

ggatttgcct atcctgggac gagtgagcgc actggttcat catcctcatc ttcatccagt     300

gcggtagtcg ccgctcggcc agaagcacca cctgcaaaag agtgtccggc cccgacgcca     360

gctgcagcag cagcggcacc gccttcagct cctgcgggta ccggaggcgg gatggtttcc     420

aaaggagagg aggataatat ggctataatt aaagagttta tgcggttcaa ggtgcatatg     480

gagggctccg taaacggtca tgagttc                                         507

<210>  8
<211>  852
<212>  DNA
<213>  artificial sequence

<220>
<223>  Hoxa13 wt gene fragment

<400>  8
cgagctctat aaaagagctc acaacccctc actcggcgcg ccagtcctcc gacagactga      60

gtcgcccggg ggggatctgg agctctcgag aattctcacg cgtcaagtgg agcaaggcag     120

gtggacagtg gatccggagc taccatggag ttgaacaaaa acatggaggg ggcggctgcg     180

gctgcagccg cggcagcggc tgcagcggct gcaggagccg gtggaggagg ttttccccat     240

ccggcggcgg ctgccgcggg ggggaatttc agtgttgcgg cagctgcagc agctgcagct     300

gctgccgcag ctaaccagtg ccgcaacctg atggcgcatc cagcgcctct tgcgccgggg     360

gccgcatcag cgtacagttc tgctcctggg gaagcacccc cgtccgcggc tgctgccgcg     420

gcagcggcag cagcagccgc ggctgcggcc gcggcagcta gctccagtgg aggtcccgga     480

ccagcgggac ctgctggtgc ggaagccgcg aagcagtgta gcccgtgcag cgcggcagct     540

cagagctcat ccggtcccgc cgccctccca tacggatact ttggctcagg gtactacccg     600

tgcgcgagaa tgggccctca ccccaacgcc ataaagtcat gtgcccaacc cgcgtcagcc     660

gcagcggcag cagcttttgc cgataagtac atggacactg ctggccccgc ggcggaggag     720

ttcagtagca gggcgaagga gggtaccgga ggcgggatgg tttccaaagg agaggaggat     780

aatatggcta taattaaaga gtttatgcgg ttcaaggtgc atatggaggg ctccgtaaac     840

ggtcatgagt tc                                                         852

<210>  9
<211>  855
<212>  DNA
<213>  artificial sequence

<220>
<223>  Hoxa13 +7A gene fragment

<400>  9

gctcacaacc cctcactcgg cgcgccagtc ctccgacaga ctgagtcgcc cgggggggat 60

ctggagctct cgagaattct cacgcgtcaa gtggagcaag gcaggtggac agtggatccg 120

gagctaccat ggagttgaac aaaaacatgg agggggcggc tgcggctgca gccgcggcag 180

cggctgcagc ggctgcagga gccggtggag gaggttttcc ccatccggcg gcggctgccg 240

cgggggggaa tttcagtgtt gcggcagctg cagcagctgc agctgctgcc gcagctaacc 300

agtgccgcaa cctgatggcg catccagcgc ctcttgcgcc gggggccgca tcagcgtaca 360

gttctgctcc tggggaagca cccccgtccg cggctgctgc cgcggcagcg gcagcagcag 420

ccgcggctgc ggcagctgca gctgcggccg ctgccgcggc agctagctcc agtggaggtc 480

ccggaccagc gggacctgct ggtgcggaag ccgcgaagca gtgtagcccg tgcagcgcgg 540

cagctcagag ctcatccggt cccgccgccc tcccatacgg atactttggc tcagggtact 600

acccgtgcgc gagaatgggc cctcacccca acgccataaa gtcatgtgcc caacccgcgt 660

cagccgcagc ggcagcagct tttgccgata agtacatgga cactgctggc cccgcggcgg 720

aggagttcag tagcagggcg aaggagggta ccggaggcgg gatggtttcc aaaggagagg 780

aggataatat ggctataatt aaagagttta tgcggttcaa ggtgcatatg gagggatctg 840

tgaacggtca cgagt 855

<210> 10
<211> 712
<212> DNA
<213> artificial sequence

<220>
<223> TBP 38Q gene fragment

<400> 10
aaagagctca caacccctca ctcggcgcgc cagtcctccg acagactgag tcgcccgggg 60

gggatctgga gctctcgaga attctcacgc gtcaagtgga gcaaggcagg tggacagtgg 120

atccggagct accatggatc agaacaacag cctgccacct tacgctcagg gcttggcctc 180

ccctcagggt gccatgactc ccggaatccc tatctttagt ccaatgatgc cttatggcac 240

tggactgacc ccacagccta ttcagaacac caatagtctg tctattttgg aagagcaaca 300

aaggcagcag cagcaacaac aacagcagca gcagcagcag cagcagcaac agcaacagca 360

gcagcagcag cagcagcagc agcagcagca gcagcagcag cagcagcagc agcaacaggc 420

agtggcagct gcagccgttc agcagtcaac gtcccagcag gcaacacagg gaacctcagg 480

ccaggcacca cagctcttcc actcacagac tctcacaact gcacccttgc cgggcaccac 540

tccactgtat ccctccccca tgactcccat gacccccatc actcctgcca cgccagcttc 600

ggagagttct gggggtaccg gaggcgggat ggtttccaaa ggagaggagg ataatatggc 660

tataattaaa gagtttatgc ggttcaaggt gcatatggag ggatctgtga ac 712

<210>  11
<211>  740
<212>  DNA
<213>  artificial sequence

<220>
<223>  TBP 53Q gene fragment

<400>  11
acccctcact cggcgcgcca gtcctccgac agactgagtc gcccgggggg gatctggagc      60

tctcgagaat tctcacgcgt caagtggagc aaggcaggtg gacagtggat ccggagctac     120

catggatcag aacaacagcc tgccacctta cgctcagggc ttggcctccc ctcagggtgc     180

catgactccc ggaatcccta tctttagtcc aatgatgcct tatggcactg gactgacccc     240

acagcctatt cagaacacca atagtctgtc tattttggaa gagcaacaaa ggcagcagca     300

gcaacaacaa cagcagcagc agcagcagca gcagcaacag caacagcagc agcagcagca     360

gcagcagcag cagcagcagc agcagcagca gcagcagcag caacagcaac agcaacagca     420

gcagcagcaa cagcagcagc agcagcaaca ggcagtggca gctgcagccg ttcagcagtc     480

aacgtcccag caggcaacac agggaacctc aggccaggca ccacagctct tccactcaca     540

gactctcaca actgcaccct tgccgggcac cactccactg tatccctccc ccatgactcc     600

catgaccccc atcactcctg ccacgccagc ttcggagagt tctgggggta ccggaggcgg     660

gatggtttcc aaaggagagg aggataatat ggctataatt aaagagttta tgcggttcaa     720

ggtgcatatg gagggatctg                                                 740


<210>  12
<211>  350
<212>  DNA
<213>  artificial sequence

<220>
<223>  Runx2 wt gene fragment

<400>  12
gcaaggcagg actagtgcca ccatggcatc aaacagcctc ttcagcacag tgacaccatg      60

tcagcaaaac ttcttttggg atccgagcac cagccggcgc ttcagccccc cctccagcag     120

cctgcagccc ggcaaaatga gcgacgtgag cccggtggtg gctgcgcaac agcagcagca     180

acagcagcag cagcaacagc agcagcagca gcagcaacag cagcagcagc agcaggaggc     240

ggcggcggcc gctgcggcgg cggcggcggc tgcggcggcg gcagctgcag tgccccggtt     300

acggccgccc cacgacaacc gcaccatggt ggagacgtac gaagccaggc                350


<210>  13
<211>  380
<212>  DNA

<213>  artificial sequence

<220>
<223>  Runx2 +10A gene fragment

<400>  13
gcaaggcagg actagtgcca ccatggcatc aaacagcctc ttcagcacag tgacaccatg      60

tcagcaaaac ttcttttggg atccgagcac cagccggcgc ttcagccccc cctccagcag      120

cctgcagccc ggcaaaatga gcgacgtgag cccggtggtg gctgcgcaac agcagcagca      180

acagcagcag cagcaacagc agcagcagca gcagcaacag cagcagcagc agcaggaggc      240

ggcggcagcc gcggctgccg cagcggctgc agcggcggcg gctgcggcgg cggcggcggc      300

tgcggcggcg gcagctgcag tgccccggtt gcggccgccc cacgacaacc gcaccatggt      360

ggagacgtac gaagccaggc                                                  380


<210>  14
<211>  211
<212>  DNA
<213>  artificial sequence

<220>
<223>  Multiple cloning site gene fragment

<400>  14
tcaagtcacg ggcgcgccag tcctccgaca gactgagtcg cccgggaaga gaggccacta      60

gtcgcgacca cgcgccggcg ggacgagacg cgatcgcgga cggcaacgta cggggtctgg      120

ctcgtcgggt ccatggtttc caaaggagag gaggataata tggctataat taaagagttt      180

atgcggttca aggtgcatat gcggaggcag g                                     211


<210>  15
<211>  39
<212>  DNA
<213>  artificial sequence

<220>
<223>  mEYFP forward primer

<400>  15
tgaggttcaa ggtgcatatg gtgagcaagg gcgaggagc                             39


<210>  16
<211>  72
<212>  DNA
<213>  artificial sequence

<220>
<223>  mEYFP reverse primer

<400>  16
aggtgggagt tgcggccgct catacctttc tcttcttttt tggaccgcct cccttgtaca      60

gctcgtccat gc                                                          72

<210>  17
<211>  48
<212>  DNA
<213>  artificial sequence

<220>
<223>  Med1 forward primer

<400>  17
gaggccgcga tcgcgagcta ccatggagca tcacagtggt agtcaggg                    48


<210>  18
<211>  59
<212>  DNA
<213>  artificial sequence

<220>
<223>  Med1 reverse primer

<400>  18
gcgaggagga taacacgtac gctccgctac ctccaagatc atcatcttcc tccccaatc        59


<210>  19
<211>  40
<212>  DNA
<213>  artificial sequence

<220>
<223>  GAL4-DBD fw primer

<400>  19
gatcgaattc atgaagctac tgtcttctat cgaacaagca                              40


<210>  20
<211>  64
<212>  DNA
<213>  artificial sequence

<220>
<223>  GAL4-DBD (short MCS) rev primer

<400>  20
gatctctaga cgtacgaccg gtgctagcga tcgccgatac agtcaactgt ctttgacctt        60

tgtt                                                                      64


<210>  21
<211>  76
<212>  DNA
<213>  artificial sequence

<220>
<223>  GAL4-DBD (long MCS) rev primer

<400>  21
gatctctaga cttaagcgta cgaccggtgc ggccgctagc gatcgccgat acagtcaact        60

gtctttgacc tttgtt                                                    76

<210>  22
<211>  32
<212>  DNA
<213>  artificial sequence

<220>
<223>  OLIG2 fw primer

<400>  22
gatcgcgatc gctttccacc cgtcggcctg cg                                  32

<210>  23
<211>  32
<212>  DNA
<213>  artificial sequence

<220>
<223>  OLIG2 rev primer

<400>  23
gatccgtacg tcacttggcg tcggaggtga gg                                  32

<210>  24
<211>  35
<212>  DNA
<213>  artificial sequence

<220>
<223>  HEY2 fw primer

<400>  24
gatcgcgatc gctcatctca gcacttgcgc caccc                               35

<210>  25
<211>  38
<212>  DNA
<213>  artificial sequence

<220>
<223>  HEY2 rev primer

<400>  25
   gatccgtacg ttaaaaagct ccaacttctg tcccccag                           38

<210>  26
<211>  543
<212>  DNA
<213>  artificial sequence

<220>
<223>  GSX2 wt sequence

<400>  26
gcgatcgctt cgcgctcctt ctatgtcgac tcgctcatca taaaggatac atcccgcccg    60

gccccgtctc tccccgaacc tcaccctggt cccgattttt ttataccctt gggaatgcct   120

ccgcccttgg tcatgtccgt gtctggtccc gggtgcccga gtaggaagag tggtgccttt 180

tgtgtttgcc cgttgtgtgt cacatcccat ctgcatagtt ctagaggcag tgtaggtgcg 240

ggttcaggcg gtgcaggtgc aggtgtaact ggagcagggg gatcaggcgt ggcgggggca 300

gccggggctc tgccgttgct caagggtcag ttcagtagcg ctccgggtga tgcccaattt 360

tgtccgagag taaatcacgc gcaccatcat caccatcccc cgcaacacca ccaccaccat 420

catcaaccac aacagcctgg atcagcggcc gccgcagcgg cagctgcggc cgcagcggct 480

gctgctgccg cgcttggaca tccacaacat cacgctccgg tatgcaccgc tacctgacgt 540

acg 543

<210> 27
<211> 495
<212> DNA
<213> artificial sequence

<220>
<223> GSX2 Ala-del sequence

<400> 27
gcgatcgctt cgcgctcctt ctatgtcgac tcgctcatca tcaaggacac ctcacggcct 60

gcgccctcgc tgcctgaacc tcaccccgga ccagacttct ttataccact cgggatgcca 120

ccacctctcg ttatgtctgt gtcaggcccc ggctgcccgt cccgcaagag cggcgcgttc 180

tgcgtgtgcc ctctctgcgt cacttcgcat ctgcactcct ctagggggag tgttggtgct 240

ggatctggtg gagctggtgc cggtgttact ggagctgggg gttcaggggt agcgggagca 300

gcgggtgctc ttcctctcct taaaggtcag ttctcttcgg ctcctgggga cgcgcagttt 360

tgcccgcggg tgaaccatgc gcatcatcat caccatcctc cgcagcacca ccatcaccat 420

catcagccac aacaaccagg ttcattggga catccacaac accacgcacc cgtatgtaca 480

gctacctgac gtacg 495

<210> 28
<211> 645
<212> DNA
<213> artificial sequence

<220>
<223> HOXD11 wt sequence

<400> 28
gcgatcgctc tggagagggc caaatggccc tatagaggtg ggggcggggg gggaagcgca 60

ggcgggggaa gtagcggtgg tggccccgga ggtggcggag gtggcgctgg gggatatgca 120

ccctattatg cagccgccgc ggcggctgct gccgcggcag ctgccgcgga agaggccgct 180

atgcaacggg aactgctccc acccgctggt agaagaccag acgttctgtt taaagcgccc 240

gaacccgttt gtgctgcccc aggcccgcct catggacctg ctggagccgc tagcaacttt 300

tactcagcag ttggacgaaa cggcatactt ccccaaggat tcgatcagtt ctatgaggcc 360

gcaccaggac cgccctttgc gggaccgcag ccaccacctc ccccagcccc tccccagcct 420

gaaggagctg cggacaaagg cgacccgaga acgggggcag gcggcggtgg aggttctccg 480

tgcacgaagg ccacaccagg gtccgaacct aaaggagccg cggagggcag cgggggggat 540

ggagaagggc cgccaggtga agccggtgca gaaaaatctt catccgcggt ggccccacaa 600

agaagccgca agaagcggtg tccatatacg aaatactgac gtacg 645

<210> 29
<211> 606
<212> DNA
<213> artificial sequence

<220>
<223> HOXD11 Ala-del sequence

<400> 29
gcgatcgctc tggaacgcgc taagtggccc tacagaggag gcggaggtgg gggatcagct 60

ggcggaggga gctcaggagg cggaccaggt ggcggtggtg gtggcgcggg tgggtacgca 120

ccctattacg aagaggcagc tatgcagcgg gagttgcttc caccagctgg gcgccgaccc 180

gatgttctgt ttaaggctcc agaacccgta tgcgctgcgc cgggaccccc gcacggtccc 240

gccggggcgg ctagtaactt ctactctgca gtgggccgca acggtatttt gccgcaaggt 300

tttgatcagt tttacgaagc agcgcccgga cctccatttg cgggcccgca accgccacca 360

cctcctgctc ctccacaacc agaaggtgcc gctgacaagg gtgaccctag gacaggtgct 420

ggtggcggcg gtggctcacc ttgtacgaag gcaacgcccg gttctgagcc caaaggtgca 480

gcggaaggta gcggcggaga cggtgaaggg ccacctggtg aggcgggagc cgagaagagt 540

tcttcagcgg tagctccaca acggtctcgc aagaagcggt gcccatatac caagtactga 600

cgtacg 606

<210> 30
<211> 609
<212> DNA
<213> artificial sequence

<220>
<223> TBX1 wt sequence

<400> 30
gcgatcgctg acccggaaga ctggcctcga aaccaccggc caggagcact cccgctgatg 60

tccgctttcg cgcggtctag gaatcccgta gcttccccta cgcaaccttc cggcaccgag 120

aaagatgcag cagaggcacg ccgggagttc cagcgggacg caggagggcc cgctgttctc 180

ggagatccgg ctcatccgcc acaacttctt gctagggtat tgtctcctag tttgcctggg 240

gcaggaggtg ctggcggcct ggtgcctctc ccaggagccc caggtgggag accatccccc 300

ccaaatccag agctgcgatt ggaagcgccc ggggcttctg aacctttgca ccaccatcca 360

tacaaatacc cagctgctgc atatgatcac taccttgggg caaaaagtag accggcaccg 420

taccccctgc ccggactgcg ggggcacgga taccatcctc acgcgcaccc acatcatcat 480

caccatcccg ttagtccggc agcggccgct gcagctgctg cggctgccgc agccgctgct 540

gcaaacatgt acagcagcgc tggagcagct cctcccggta gttatgatta ctgccccaga 600

tgacgtacg 609

<210> 31
<211> 555
<212> DNA
<213> artificial sequence

<220>
<223> TBX1 Ala-del sequence

<400> 31
   gcgatcgctg accctgaaga ctggccgagg aaccataggc caggcgcact cccccttatg 60
   agtgccttcg ctcgatccag aaatccggtc gcaagtccaa cacaaccatc tggaaccgag 120
   aaagatgcag ctgaagcacg acgggagttc cagcgagatg ccgggggacc tgcagtactg 180
   ggtgatcccg cacatcctcc ccaacttttg gcgagagtat tgagtccctc ccttcctggt 240
   gctggaggag ctggcggtct cgtaccactt ccaggagcgc cgggtggtcg gccatccccg 300
   cctaacccag aactccgact tgaggctcca ggcgcttccg aaccgctgca tcatcatccg 360
   tacaagtacc cttacgatca ttacctgggc gctaagagca gaccggctcc gtaccctctg 420
   ccgggcctca gaggacatgg gtatcaccct cacgcgcacc cgcaccacca tcaccatcct 480
   gtgtcaccga acatgtattc cagcgcgggg gccgctcctc ctggtagcta cgattactgt 540
   cctagatgac gtacg 555

<210> 32
<211> 738
<212> DNA
<213> artificial sequence

<220>
<223> EOMES wt sequence

<400> 32
gcgatcgcta acctgcctgg cgcgcacttc tacccgctgg agagcgctcg cggaggtagt 60

gggggctctg cgggccacct tccctccgct gcgccatcac cgcaaaaact tgacctcgat 120

aaggctagta aaaaattttc aggctctctg tcatgtgaag cggtcagcgg ggaaccggcg 180

gcagcctccg ctggtgcacc agccgcgatg ttgtcagata cggatgctgg cgacgctttt 240

gcgagtgctg ctgctgtagc aaaacctggc cctcctgatg ggagaaaagg ctctccatgc 300

ggagaggagg aacttccaag tgccgcagct gcagcagctg cagcggcagc agcagcggct 360

gccacggcac ggtattctat ggattcactt tcctctgaaa gatactacct ccagtctcct 420

ggcccacaag ggtctgagct cgccgcccct tgttcattgt tcccctacca ggcagccgca 480

ggcgctccac acggcccggt ttatcctgct cccaatggtg caagataccc atatgggagt 540

atgttgccac ctggaggctt cccggcggcc gtctgtccac cagggcgggc tcagtttgga 600

cctggagcag gtgctggtag tggggctggt ggttcctcag gagggggtgg cggtcctggt 660

acttaccaat acagccaagg agccccgctc tatgggccgt accctggagc cgcagcggcg 720

ggatcttgct gacgtacg 738

<210> 33
<211> 816
<212> DNA
<213> artificial sequence

<220>
<223> BHLHE41 wt sequence

<400> 33
gcgatcgctt gcgtccctgt catacaaagg acgcaaccct ctgcggagtt ggccgcggaa 60

aatgacacag acaccgattc aggttatggt ggcgaggcag aggcgcgccc cgatagggaa 120

aagggcaaag gcgcgggcgc atctagggtc acaataaagc aagagccacc aggagaggat 180

tcaccagccc cgaagaggat gaaattggac tccagaggtg gcgggtcagg aggaggaccg 240

ggcggcggag ctgcagccgc cgcagctgct cttttgggac cagatccagc cgcggccgca 300

gcccttctta gacccgacgc ggctctcctc tcctctctgg tcgcatttgg cgggggaggt 360

ggagcccctt tcccccaacc agctgccgcg gcagcaccct tttgtctccc cttctgcttc 420

ctgtctccta gcgccgcagc agcctatgtg caacctttcc tggacaagtc tggtcttgaa 480

aaatatctct acccagcagc tgccgcagct ccatttccac tgttgtatcc gggaatccca 540

gccccagctg cggccgctgc cgccgcagca gcggctgctg cagcagcagc tgcgttccca 600

tgcctctcct ctgttcttag tcctcctcca gagaaggcag gagctgccgc cgctacactg 660

ctcccccatg aggttgctcc tcttggagcc cctcatccgc aacacccaca cggtaggaca 720

caccttccat ttgctgggcc aagagagcca gggaatccag agagcagcgc acaggaagac 780

cctagtcagc ctggcaagga ggccccctga cgtacg 816

<210> 34
<211> 678
<212> DNA
<213> artificial sequence

<220>

<223>  BHLHE41 Ala-del sequence

<400>  34
gcgatcgctt gcgtgcccgt catccagcgg actcagccct ccgcagagct ggccgctgaa      60

aatgatactg ataccgactc cggctacgga ggtgaagctg aggcaaggcc ggaccgggag     120

aaaggaaaag gcgcgggtgc ttcccgcgtc acaataaaac aggagccccc aggagaagac     180

tcacctgcac ctaagaggat gaagctggat tccaggggag gaggttctgg aggcggaccc     240

ggaggcggcc tgctgggccc agaccctttg ctcaggcctg atgcggcgct gttgtccagt     300

cttgtcgcat ttggaggcgg gggaggagca ccattcccac agccaccgtt ctgtctccct     360

ttttgttttc tgtcacctag ttatgttcaa ccttttcttg ataagagcgg actcgaaaag     420

tatctgtatc caccgttccc acttctgtat cctggaatcc ccgcgccctt tccatgcctg     480

agcagtgtat tgtctccgcc ccccgagaaa gctggcaccc tgttgccgca tgaggtggct     540

ccgctgggag cacctcaccc acaacatccg cacggtagga ctcacttgcc tttcgctggc     600

ccccgggaac ctgggaaccc cgagtctagc gctcaagagg atcccagcca acccggcaag     660

gaggctccct gacgtacg                                                   678


<210>  35
<211>  438
<212>  DNA
<213>  artificial sequence

<220>
<223>  HOXA13 -58A (Ala-del) sequence

<400>  35
gcgatcgctg agttgaacaa aaacatggag gggggagccg gtggaggagg ttttccccat      60

ccggggggga atttcagtgt taaccagtgc cgcaacctga tggcgcatcc agcgcctctt     120

gcgccggggg ccgcatcagc gtacagttct gctcctgggg aagcaccccc gtccagcagt     180

tctggcggac cagggcccgc aggccctgct ggtgctgagg cggcgaaaca gtgcagcccc     240

tgttctcaga gctcatccgg tcccgccgcc ctcccatacg gatactttgg ctcaggttac      300

tacccgtgcg cgagaatggg ccctcacccc aacgccataa agtcatgtgc ccaacccgcg     360

tcatttgccg ataagtacat ggacactgct ggccccgcgg cggaggagtt cagtagcagg     420

gcgaaggagt gacgtacg                                                   438


<210>  36
<211>  480
<212>  DNA
<213>  artificial sequence

<220>
<223>  HOXA13 -44A sequence

<400>  36

gcgatcgctg agttgaacaa aaacatggag gggggggctg gaggaggtgg cttcccgcat 60

cctgctgctg cggcagctgg ggggaatttt tcagtcaatc agtgcagaaa cctgatggcg 120

caccctgccc cacttgcacc gggagctgca tcagcgtaca gctcagcgcc gggagaagct 180

ccaccgagct catctagtgg tggacccgga ccagcgggtc ccgctggggc cgaggctgcg 240

aaacaatgtt ccccctgttc tgcagccgca cagtcctctt caggccctgc ggcattgcca 300

tatggatact ttgggtcagg ctattatccc tgcgcccgca tgggtcctca tccaaatgcg 360

atcaagagct gcgcgcagcc tgctagtgcc gcggcagcgg cagcctttgc tgataaatac 420

atggataccg cgggccccgc agcagaagaa tttagcagcc gagctaagga gtgacgtacg 480

<210> 37
<211> 585
<212> DNA
<213> artificial sequence

<220>
<223> MNX1 wt sequence

<400> 37
gcgatcgctg cggttgatcc tcctagagct gcgagtgctc agagtgcccc cttggccttg 60

gttacgagtc tggcagctgc tgctagcggc acgggtgggg gaggcggcgg tgggggagct 120

agtgggggga catcaggctc atgctctcca gcttcctccg agccgcctgc cgctcccgcc 180

gataggctcc gagcagagag tccatcaccg cctcggctgt tggcagccca ctgtgctttg 240

cttcccaaac cagggttttt gggcgctggt ggcggagggg gaggaacggg aggtggtcac 300

ggcggccctc atcaccatgc tcatccgggt gccgctgcgg ctgctgcggc agcagctgcg 360

gcggcagccg caggtggtct tgcactcggt cttcacccgg gcggagcaca aggtggcgcg 420

ggacttcctg cacaagcagc tctgtatggg cacccagtct atgggtatag tgcggcggct 480

gcggcagccg cccttgcagg gcaacaccca gccttgtctt attcttatcc tcaggtacag 540

ggggcgcatc cagcacaccc ggcggacccg ataaagtgac gtacg 585

<210> 38
<211> 510
<212> DNA
<213> artificial sequence

<220>
<223> MNX1 Ala-del sequence

<400> 38
gcgatcgctg cggtggaccc cccacgagcc gcctcagcgc aatctgcgcc attggctctg 60

gtcacttcat tgtcaggaac aggaggtggg ggtggaggag gaggtgcgtc aggcggaacg 120

agcgggagct gttcaccggc ctcatccgaa cctccggcgg cacccgccga cagacttcgg 180

gctgaaagcc cgtccccccc acggctcctg gctgcgcatt gtgctctgtt gcctaaaccg 240

ggatttctgg gtgcaggtgg tgggggaggg ggtacaggcg gaggtcacgg cggcccacac 300

catcatgctc accctggagg cgggttggcg ctcggcctcc atcccggggg tgcccaaggt 360

ggggctggat tgccagcaca agcggctctg tatggacatc cggtatatgg ctattctctt 420

gctggacagc accctgctct gtcctatagt taccctcagg tacagggggc ccatccagcg 480

catcccgcag accctatcaa gtgacgtacg 510

<210> 39
<211> 498
<212> DNA
<213> artificial sequence

<220>
<223> HEY2 Ala-del sequence

<400> 39
gcgatcgctc atctcagcac ttgcgccacc cagcgggaga tgacatcctc catggcccac 60

caccatcatc cgctccaccc gcatcactgg ttccaccacc tgcccgcagc cctgctccag 120

cccaacggcc tccatgcctc agagtcaacc ccttgtcgcc tctccacaac ttcagaagtg 180

cctcctgccc acggctctgc tctcctcacg gccacgtttg cccatgcgga ttcagccctc 240

cgaatgccat ccacgggcag cgtcgccccc tgcgtgccac ctctctccac ctctctcttg 300

tccctctctg ccaccgtcca cacccacagc ttccctctgt ccttcgcggg ggcattcccc 360

atgcttcccc caaacgtgac agccatcagc ccgcccttgt cagtatcagc cacgtccagt 420

cctcagcaga ccagcagtgg aacaaacaat aaaccttacc gaccctgggg gacagaagtt 480

ggagcttttt aacgtacg 498

<210> 40
<211> 372
<212> DNA
<213> artificial sequence

<220>
<223> OLIG2 Ala-del sequence

<400> 40
   gcgatcgctt tccatccctc tgcttgcgga ggtctggcgc acagcgcacc tttgcccgct 60
   gccacggcac atccacatgc ggcacatcat ccggccgtgc accatcctat actcccgccc 120
   gtatctagcg catcattgcc ggggagtggc ctccctagtg taggttccat cagaccacca 180
   catggacttc ttaaatcccc gtcccccctt ggtggtggtg ggggtggatc tggagcgtcc 240
   ggaggctttc aacattgggg cggaatgcca tgcccgtgtt ccatgtgcca agtcccccct 300
   ccacatcacc acgtctctgc aatgggtgct gggtcactcc ctaggctcac tagcgacgcc 360
   aagtagcgta cg 372

<210>  41
<211>  56
<212>  DNA
<213>  artificial sequence

<220>
<223>  MCS fw primer

<400>  41
gatcggatcc gcgatcgcta gcaccggtcg tacgtctaga gggcccgaat tcgatc          56


<210>  42
<211>  56
<212>  DNA
<213>  artificial sequence

<220>
<223>  MCS rev primer

<400>  42
gatcgaattc gggccctcta gacgtacgac cggtgctagc gatcgcggat ccgatc          56

## Description

The present invention relates to a method of determining the capacity of a Cluster 1 mammalian Transcription Factor TF) for phase separation and/or the capacity for forming a transcriptional condensate in a sample. Further, the present invention relates to an active agent for use in a method of preventing and/or treating a disorder associated with, caused by and/or accompanied with a dysfunction of a biomolecular condensate comprising at least one Cluster 1 mammalian TF.

More than 30 inherited human disorders are caused by an abnormal expansion of short, repetitive DNA sequence elements (Albrecht and Mundlos, 2005; Darling and Uversky, 2017; La Spada and Taylor, 2010; Orr and Zoghbi, 2007). The majority (>20) of such repeat expansions occur in protein coding genes and lead to expansions of homopolymeric alanine or glutamine repeats in cellular proteins. Glutamine repeat expansions are typically associated with devastating neurodegenerative diseases e.g. Huntington's disease and spinocerebellar ataxia (La Spada and Taylor, 2010; Orr and Zoghbi, 2007), and alanine repeat expansions are typically associated with severe developmental disorders e.g. X-linked retardation, congenital ventral hypoventilation and synpolydactyly (Albrecht and Mundlos, 2005).

Virtually all investigations to date have focused on three features of proteins that contain disease-associated repeat expansions, as cause of their pathology: their proclivity to form solid aggregates, alteration in their subcellular localization, and alteration of their proteolytic processing (Darling and Uversky, 2017; Orr and Zoghbi, 2007; Ross and Poirier, 2004). For example, many studies have established a correlation between aggregate formation by the mutant HTT protein and degeneration of specific neurons in Huntington's disease (Davies et al., 1997; Ross and Poirier, 2004; Zoghbi and Orr, 2000). However, several lines of evidence suggest that perturbed function of the soluble, appropriately localized fraction of the repeat-expanded protein may be responsible for its pathological effect, rather than aggregates (Ross, 2002; Saudou et al., 1998; Truant et al., 2008). Improved understanding of the function of the affected proteins and how the repeat expansions interfere with those functions would facilitate the development of therapeutics for this family of disease.

The majority (15/20) of disease-associated repeat expansions occur in nuclear proteins, most of which are sequence-specific transcription factors (TFs) (Darling and Uversky, 2017). For example, expansions of an alanine repeat in the homeobox TF HOXD13 cause synpolydactyly, a hereditary limb malformation disorder (Muragaki et al., 1996), and expansions of a glutamine repeat in the highly conserved transcription factor TATA-box binding protein (TBP) causes spinocerebellar ataxia type 17, a progressive neurodegenerative disease (Nakamura et al., 2001). New insights into how TFs interact with components of the transcription machinery to control gene expression programs would thus likely afford significant advance in our understanding of how repeat expansions in transcriptional regulators lead to pathology.

Control of gene transcription in eukaryotes involves the recruitment of RNA Polymerase II (RNAPII) to genomic sites by sequence-specific TFs assisted by diverse transcriptional co-activators (Levine et al., 2014). Separation of liquids into a dense and dilute phase underlies the formation of several subcellular membraneless organelles (Alberti et al., 2019; Banani et al., 2017; Shin and Brangwynne, 2017), and recent evidence suggests that the assembly of the transcription machinery at genomic sites occurs through liquid-liquid phase separation, leading to the formation of transcriptional condensates (Boehning et al., 2018; Chong et al., 2018; Hnisz et al., 2017; Li et al., 2019; Sabari et al., 2018). For example, several TFs (e.g. FET family TFs, OCT4, SP1), co-activators (e.g. Mediator, BRD4) and RNA Polymerase II contain intrinsically disordered regions (IDRs) that drive their phase separation, and these factors form discrete nuclear puncta in mammalian cells (Boehning et al., 2018; Cho et al., 2018; Chong et al., 2018; Kwon et al., 2013; Lu et al., 2018; Sabari et al., 2018). While TF- and co-activator-containing condensates are sensitive to short-chain aliphatic alcohols that dissolve various intracellular membraneless organelles (Boehning et al., 2018; Chong et al., 2018; Sabari et al., 2018), the functional importance of phase separation in transcriptional control has been unclear.

In the present application, we describe the molecular features that drive condensate formation of human TFs. We developed a clustering algorithm to classify human TFs based on sequence features of their intrinsically disordered regions (IDRs), and applied this algorithm to the ∼1,500 TFs found in humans. The classification algorithm revealed 7 different types of IDRs in human TFs.

We also provide evidence that for a class of TFs in humans, the molecular feature that drives condensate formation and transcriptional activity is driven by hydrophobicity (Cluster 1 TFs).

Further, we provide evidence that disease-mutations that cause a spectrum of genetic diseases in humans are highly enriched among the class of Cluster 1 TFs, and that disease-mutations in those TFs perturb the composition of condensates formed by those factors in vitro and in vivo.

Furthermore, we provide several lines of experimental evidence that disease-associated mutations in Cluster 1 TFs perturb (1) the condensation capacity of those factors, (2) the composition of condensates formed by those factors, and (3) the transcriptional activity of those factors:
(1) Disease-associated mutations in the Hoxd13 TF enhanced its condensation in an optogenetic system in live cells, and the enhanced condensation of purified, recombinant Hoxd13 in vitro Disease-associated mutations in the Hoxa13 TF enhanced its condensation in an optogenetic system in live cells and the enhanced condensation of purified, recombinant Hoxa13 in vitro. Disease-associated mutations in the Runx2 TF enhanced its condensation in an optogenetic system in live and the enhanced condensation of purified, recombinant Runx2 in vitro.
(2) Disease-associated mutations in the Hoxd13 TF led to reduction of the transcriptional co-activator content of Hoxd13-containing condensates in vitro and in cells. Disease-associated mutations in the Hoxa13 TF led to reduction of the transcriptional co-activator content of Hoxa13-containing condensates in vitro. Disease-associated mutations in the Runx2 TF led to reduction of the transcriptional co-activator content of Runx2-containing condensates in vitro.
(3) Disease-associated mutations reduced the transcriptional activity of Hoxd13, Hoxa13, and Runx2.

Furthermore, we demonstrate that amphiphilic small molecules such as ATP can rescue the composition of condensates formed by Cluster 1 TFs containing disease-mutations. ATP (adenosine tri-phosphate) is a small amphiphilic molecule that consist of a hydrophobic base, and a hydrophilic tail. Treatment of heterotypic condensates formed by Cluster 1 TFs with transcriptional co-activators led to an increase of co-activator content and a decrease of TF content. ATP treatment thus was found to revert the compositional alteration of disease-associated mutation in Cluster 1 TFs. This effect is at least in part specific to ATP, as other small molecules that are known to dissolve condensates formed by cytosolic (non-hydrophobic) stress granule proteins were ineffective.

The methods described here are broadly applicable to modulate the composition of biomolecular condensates formed by Cluster 1 human TFs, and infer biological activity based on the condensate composition. Further, amphiphilic small molecules with drug-like features such as ATP can modulate the composition of biomolecular condensates formed by Cluster 1 human TFs. Furthermore, the link between transcriptional activity and condensate composition described here can be used to detect and measure condensate composition for prognostic and diagnostic applications.

According to the present invention, methods for determining and/or modulating the composition and/or activity of a Cluster 1 mammalian Transcription Factor (TF) and/or a transcriptional condensate comprising at least one Cluster 1 mammalian TF are provided.

A first aspect of the present invention relates to a method of determining the capacity of a Cluster 1 mammalian TF for phase separation and/or the capacity for forming a transcriptional condensate.

The capacity for phase separation and/or the capacity for forming a transcriptional condensate may be determined on an isolated Cluster 1 TF, e.g. a TF from a natural source or a recombinantly produced TF, optionally in the presence of further components of a transcriptional condensate or in a cellular context, e.g. in a cell or cell extract sample comprising the Cluster 1 TF. In certain embodiments, the sample is derived from a mammalian subject, e.g. a human subject.

A further aspect of the present invention relates to an amphiphilic molecule comprising (i) a hydrophobic component and a (ii) a hydrophilic component for use in a method of preventing and/or treating a disorder associated with, caused by and/or accompanied with a dysfunction of a biomolecular condensate comprising at least one Cluster 1 mammalian TF.

Still a further aspect of the present invention relates to a method of preventing and/or treating a disorder associated with, caused by and/or accompanied with a dysfunction of a biomolecular condensate comprising at least one Cluster 1 mammalian TF, comprising administering to a subject in need thereof, particularly a human subject, a therapeutically effective amount of an amphiphilic molecule comprising (i) a hydrophobic component and (ii) a hydrophilic component.

According to the present invention, it was found that the presence of a functional mutation, particularly of a disease-associated mutation in a Cluster 1 TF can be identified by determining its capacity of phase separation and/or by determining its capacity for forming a transcriptional condensate, e.g. its condensation capacity, the composition of a transcriptional condensate comprising such a factor, and/or the transcriptional activity of the TF or a condensate comprising the TF.

In certain embodiments, the TF is a Cluster 1 human TF particularly selected from the group of human Transcription Factors shown in Table 1:

| **Transcription Factor** | **GenBank_ID** | **Uniprot_ID** |
|---|---|---|
| BCL11B | 64919 | Q9C0K0 |
| EGR2 | 1959 | P11161 |
| EGR4 | 1961 | Q05215 |
| FEZF1 | 389549 | A0PJY2 |
| FEZF2 | 55079 | Q8TBJ5 |
| FIZ1 | 84922 | Q96SL8 |
| GLI4 | 2738 | P10075 |
| GLIS1 | 148979 | Q8NBF1 |
| GLIS2 | 84662 | Q9BZE0 |
| HIC1 | 3090 | Q14526 |
| INSM1 | 3642 | Q01101 |
| INSM2 | 84684 | Q96T92 |
| KLF1 | 10661 | Q13351 |
| KLF14 | 136259 | Q8TD94 |
| KLF16 | 83855 | Q9BXK1 |
| KLF2 | 10365 | Q9Y5W3 |
| KLF4 | 9314 | 043474 |
| MAZ | 4150 | P56270 |
| PRDM12 | 59335 | Q9H4Q4 |
| PRDM13 | 59336 | Q9H4Q3 |
| PRDM14 | 63978 | Q9GZV8 |
| PRDM6 | 93166 | Q9NQX0 |
| PRDM8 | 56978 | Q9NQV8 |
| SALL3 | 27164 | Q9BXA9 |
| SCRT1 | 83482 | Q9BWW7 |
| SCRT2 | 85508 | Q9NQ03 |
| SLC2A4RG | 56731 | Q9NR83 |
| SNAI3 | 333929 | Q3KNW1 |
| SP5 | 389058 | Q6BEB4 |
| SP6 | 80320 | Q3SY56 |
| SP7 | 121340 | Q8TDD2 |
| SP8 | 221833 | Q8IXZ3 |
| SP9 | 100131390 | P0CG40 |
| WIZ | 58525 | O95785 |
| WT1 | 7490 | P19544 |
| ZBTB42 | 100128927 | B2RXF5 |
| ZBTB45 | 84878 | Q96K62 |
| ZBTB7C | 201501 | A1YPR0 |
| ZIC2 | 7546 | 095409 |
| ZIC3 | 7547 | 060481 |
| ZIC5 | 85416 | Q96T25 |
| ZNF219 | 51222 | Q9P2Y4 |
| ZNF281 | 23528 | Q9Y2X9 |
| ZNF335 | 63925 | Q9H4Z2 |
| ZNF367 | 195828 | Q7RTV3 |
| ZNF385A | 25946 | Q96PM9 |
| ZNF385C | 201181 | Q66K41 |
| ZNF395 | 55893 | Q9H8N7 |
| ZNF414 | 84330 | Q96IQ9 |
| ZNF444 | 55311 | Q8N0Y2 |
| ZNF469 | 84627 | Q96JG9 |
| ZNF503 | 84858 | Q96F45 |
| ZNF513 | 130557 | Q8N8E2 |
| ZNF668 | 79759 | Q96K58 |
| ZNF683 | 257101 | Q8IZ20 |
| ZNF703 | 80139 | Q9H7S9 |
| ZNF784 | 147808 | Q8NCA9 |
| ZNF787 | 126208 | Q6DD87 |
| ZNF843 | 283933 | Q8N446 |
| ZNF865 | 100507290 | P0CJ78 |
| ZXDA | 7789 | P98168 |
| ZXDB | 158586 | P98169 |
| ZNF316 | 100131017 | A6NFI3 |
| ZNF517 | 340385 | Q6ZMY9 |
| ZNF534 | 147658 | Q76KX8 |
| ZNF74 | 7625 | Q16587 |
| ZNF746 | 155061 | Q6NUN9 |
| ALX3 | 257 | O95076 |
| ARX | 170302 | Q96QS3 |
| BARX1 | 56033 | Q9HBU1 |
| BSX | 390259 | Q3C1V8 |
| CDX1 | 1044 | P47902 |
| CDX2 | 1045 | Q99626 |
| DBX2 | 440097 | Q6ZNG2 |
| DLX4 | 1748 | Q92988 |
| DUX4 | 100288687 | Q9UBX2 |
| EMX1 | 2016 | Q04741 |
| EMX2 | 2018 | Q04743 |
| EN1 | 2019 | Q05925 |
| EN2 | 2020 | P19622 |
| ESX1 | 80712 | Q8N693 |
| EVX2 | 344191 | Q03828 |
| GBX1 | 2636 | Q14549 |
| GBX2 | 2637 | P52951 |
| GSC2 | 2928 | O15499 |
| GSX1 | 219409 | Q9H4S2 |
| GSX2 | 170825 | Q9BZM3 |
| HMX1 | 3166 | Q9NP08 |
| HOXA10 | 3206 | P31260 |
| HOXA13 | 3209 | P31271 |
| HOXA4 | 3201 | Q00056 |
| HOXB13 | 10481 | Q92826 |
| HOXB2 | 3212 | P14652 |
| HOXB4 | 3214 | P17483 |
| HOXC12 | 3228 | P31275 |
| HOXC13 | 3229 | P31276 |
| HOXC5 | 3222 | Q00444 |
| HOXD1 | 3231 | Q9GZZ0 |
| HOXD11 | 3237 | P31277 |
| HOXD12 | 3238 | P35452 |
| HOXD13 | 3239 | P35453 |
| HOXD3 | 3232 | P31249 |
| HOXD4 | 3233 | P09016 |
| HOXD9 | 3235 | P28356 |
| IRX1 | 79192 | P78414 |
| IRX4 | 50805 | P78413 |
| ISL2 | 64843 | Q96A47 |
| LBX2 | 85474 | Q6XYB7 |
| LHX2 | 9355 | P50458 |
| LHX3 | 8022 | Q9UBR4 |
| LHX6 | 26468 | Q9UPM6 |
| MIXL1 | 83881 | Q9H2W2 |
| MNX1 | 3110 | P50219 |
| MSX1 | 4487 | P28360 |
| MSX2 | 4488 | P35548 |
| NOBOX | 135935 | O60393 |
| OTP | 23440 | Q5XKR4 |
| PBX4 | 80714 | Q9BYU1 |
| PDX1 | 3651 | P52945 |
| PHOX2A | 401 | O14813 |
| PHOX2B | 8929 | Q99453 |
| PITX3 | 5309 | 075364 |
| PRRX2 | 51450 | Q99811 |
| RAX | 30062 | Q9Y2V3 |
| RAX2 | 84839 | Q96IS3 |
| SEBOX | 645832 | Q9HB31 |
| TGIF1 | 7050 | Q15583 |
| TLX1 | 3195 | P31314 |
| TLX2 | 3196 | O43763 |
| TLX3 | 30012 | 043711 |
| TPRX1 | 284355 | Q8N7U7 |
| UNCX | 340260 | A6NJT0 |
| VAX1 | 11023 | Q5SQQ9 |
| VAX2 | 25806 | Q9UIW0 |
| VSX1 | 30813 | Q9NZR4 |
| ZFHX2 | 85446 | Q9C0A1 |
| AHRR | 57491 | A9YTQ3 |
| ASCL2 | 430 | Q99929 |
| BHLHA9 | 727857 | Q7RTU4 |
| BHLHE22 | 27319 | Q8NFJ8 |
| BHLHE23 | 128408 | Q8NDY6 |
| BHLHE41 | 79365 | Q9C0J9 |
| HAND1 | 9421 | 096004 |
| HELT | 391723 | A6NFD8 |
| HES2 | 54626 | Q9Y543 |
| HES3 | 390992 | Q5TGS1 |
| HES6 | 55502 | Q96HZ4 |
| HES7 | 84667 | Q9BYE0 |
| HEY1 | 23462 | Q9Y5J3 |
| HEY2 | 23493 | Q9UBP5 |
| HEYL | 26508 | Q9NQ87 |
| HIF3A | 64344 | Q9Y2N7 |
| MESP1 | 55897 | Q9BRJ9 |
| MESP2 | 145873 | Q0VG99 |
| MLXIPL | 51085 | Q9NP71 |
| MSGN1 | 343930 | A6NI15 |
| OLIG2 | 10215 | Q13516 |
| OLIG3 | 167826 | Q7RTU3 |
| PTF1A | 256297 | Q7RTS3 |
| SIM2 | 6493 | Q14190 |
| SOHLH1 | 402381 | Q5JUK2 |
| TAL1 | 6886 | P17542 |
| TCF3 | 6929 | P15923 |
| TCFL5 | 10732 | Q9UL49 |
| BATF2 | 116071 | Q8N1L9 |
| CEBPA | 1050 | P49715 |
| CEBPB | 1051 | P17676 |
| CEBPD | 1052 | P49716 |
| CEBPE | 1053 | Q15744 |
| DBP | 1628 | Q10586 |
| JUNB | 3726 | P17275 |
| JUND | 3727 | P17535 |
| MAF | 4094 | O75444 |
| MAFA | 389692 | Q8NHW3 |
| NRL | 4901 | P54845 |
| FOXA1 | 3169 | P55317 |
| FOXA2 | 3170 | Q9Y261 |
| FOXA3 | 3171 | P55318 |
| FOXB2 | 442425 | Q5VYV0 |
| FOXC1 | 2296 | Q12948 |
| FOXC2 | 2303 | Q99958 |
| FOXD1 | 2297 | Q16676 |
| FOXD2 | 2306 | O60548 |
| FOXD3 | 27022 | Q9UJU5 |
| FOXD4 | 2298 | Q12950 |
| FOXD4L1 | 200350 | Q9NU39 |
| FOXE1 | 2304 | 000358 |
| FOXE3 | 2301 | Q13461 |
| FOXH1 | 8928 | O75593 |
| FOXI2 | 399823 | Q6ZQN5 |
| FOXJ1 | 2302 | Q92949 |
| FOXK1 | 221937 | P85037 |
| FOXK2 | 3607 | Q01167 |
| FOXL1 | 2300 | Q12952 |
| FOXL2 | 668 | P58012 |
| FOXN1 | 8456 | O15353 |
| FOXN4 | 121643 | Q96NZ1 |
| FOXO6 | 100132074 | A8MYZ6 |
| FOXP3 | 50943 | Q9BZS1 |
| FOXQ1 | 94234 | Q9C009 |
| FOXS1 | 2307 | 043638 |
| AR | 367 | P10275 |
| NR2F1 | 7025 | P10589 |
| NR5A1 | 2516 | Q13285 |
| PGR | 5241 | P06401 |
| RXRB | 6257 | P28702 |
| CIC | 23152 | Q96RK0 |
| SOX1 | 6656 | 000570 |
| SOX15 | 6665 | 060248 |
| SOX17 | 64321 | Q9H6I2 |
| SOX18 | 54345 | P35713 |
| SOX21 | 11166 | Q9Y651 |
| SOX3 | 6658 | P41225 |
| SOX4 | 6659 | Q06945 |
| TCF7 | 6932 | P36402 |
| POU3F1 | 5453 | Q03052 |
| POU3F2 | 5454 | P20265 |
| POU3F3 | 5455 | P20264 |
| POU4F1 | 5457 | Q01851 |
| POU5F1 | 5460 | Q01860 |
| POU5F1B | 5462 | Q06416 |
| POU5F2 | 134187 | Q8N7G0 |
| GATA1 | 2623 | P15976 |
| GATA2 | 2624 | P23769 |
| GATA4 | 2626 | P43694 |
| GATA5 | 140628 | Q9BWX5 |
| GATA6 | 2627 | Q92908 |
| GLMP | 112770 | Q8WWB7 |
| PURA | 5813 | Q00577 |
| SKOR1 | 390598 | P84550 |
| SNAPC2 | 6618 | Q13487 |
| XPA | 7507 | P23025 |
| TRIM28 | 10155 | Q13263 |
| ARID3C | 138715 | A6NKF2 |
| AHDC1 | 27245 | Q5TGY3 |
| DOT1L | 84444 | Q8TEK3 |
| PRR12 | 57479 | Q9ULL5 |
| ZNF541 | 84215 | Q9H0D2 |
| TIGD5 | 84948 | Q53EQ6 |
| ONECUT2 | 9480 | 095948 |
| ONECUT3 | 390874 | 060422 |
| DMRT1 | 1761 | Q9Y5R6 |
| DMRTA1 | 63951 | Q5VZB9 |
| DMRTA2 | 63950 | Q96SC8 |
| DMRTB1 | 63948 | Q96MA1 |
| DMRTC2 | 63946 | Q81XT2 |
| E2F2 | 1870 | Q14209 |
| E2F3 | 1871 | 000716 |
| ERF | 2077 | P50548 |
| ETS2 | 2114 | P15036 |
| ETV2 | 2116 | 000321 |
| ETV3L | 440695 | Q6ZN32 |
| FEV | 54738 | Q99581 |
| SPDEF | 25803 | 095238 |
| SPIB | 6689 | Q01892 |
| MEF2B | 100271849 | Q02080 |
| MYPOP | 339344 | Q86VE0 |
| SNAPC4 | 6621 | Q5SXM2 |
| NFATC2 | 4773 | Q13469 |
| NFATC4 | 4776 | Q14934 |
| RELB | 5971 | Q01201 |
| RFX5 | 5993 | P48382 |
| AIRE | 326 | 043918 |
| EOMES | 8320 | 095936 |
| TBX1 | 6899 | 043435 |
| TBX10 | 347853 | 075333 |
| TBX2 | 6909 | Q13207 |
| TBX3 | 6926 | 015119 |
| TBX6 | 6911 | O95947 |
| RUNX2 | 860 | Q13950 |

Table 1 shows the names of the respective Cluster 1 TFs and their GenBank and UniProt identification numbers. It should be noted, however, that the invention also encompasses a functional variant of any of these Cluster 1 TFs, including an allelic variant, a wild-type variant or disease-associated variant, and a recombinant variant, e.g. a variant fused to a heterologous peptide or polypeptide. Particularly, the invention also encompasses a disease-associated variant of a Cluster 1 TF, particularly a disease-associated variant comprising an amino acid repeat expansion as described herein in detail, infra, wherein the disease-associated variant is optionally fused to a heterologous peptide or polypeptide.

More particularly, the Cluster 1 human TF is selected from the group consisting of HOXD13 (GenBank_ID: 3239; UniProt_ID: P35453), HOXA13 (GenBank_ID: 3209; UniProt_ID: P31271), RUNX2 (GenBank_ID: 860; UniProt_ID: Q13950), SOX3 (GenBank_ID: 6658; UniProt_ID: P41225), FOXL2 (GenBank_ID: 668; UniProt_ID: P58012), ZIC2 (GenBank_ID: 7546; UniProt_ID: O95409), ARX (GenBank_ID: 170302; UniProt_ID: Q96QS3), PHOX2B (GenBank_ID: 8929; UniProt_ID: Q99453), and AR (GenBank_ID: 367; UniProt_ID: P10275) including a functional variant of any of these TFs, more particularly a disease-associated variant comprising an amino acid repeat expansion, which is associated with a genetic disorder.

TFs, e.g. Cluster 1 TFs form transcriptional condensates through the biophysical process called phase separation. Phase separation is mediated through intrinsically disordered regions (IDRs) found in those proteins. Further proteins in transcriptional condensates include transcriptional co-activators e.g. the Mediator complex, or sub-units thereof, the chromatin reader BRD4, RNA Polymerase II (RNAPII), FET-family TFs, and additional TFs involved in extracellular signaling. These condensates are herein referred to as transcriptional condensates. Transcriptional condensates tend to have heterotypic composition in vivo.

The present inventors have found that an altered capacity of a Cluster 1 TF for phase separation and/or for forming a transcriptional condensate versus the wild-type TF is indicative for the presence of a disease-associated mutation in the Cluster 1 TF. A disease-associated mutation may comprise an expansion of an amino acid repeat in an intrinsically disordered region (IDR) of the respective TF. In certain embodiments, the expansion comprises an expansion of at least 2, 3, 4, 5, 6, 7, 8, 9, 10, 11, 12, 13, 14, 15 or more amino acid residues, particularly hydrophobic amino acid residues, including but not limited to alanine, in the IDR of the TF.

The present invention encompasses determining the capacity of a Cluster 1 mammalian TF for phase separation and/or for forming a transcriptional condensate. The determination may be carried out in a sample comprising the cluster 1 TF and optionally further components. The sample may be a biological sample selected from a body fluid sample, e.g. blood, serum, plasma, urine, saliva etc., a tissue sample, e.g. a tissue section, a tissue homogenate, a tissue extract, a cell culture sample, e.g. a sample of cultured cells or an extract from cultured cells, or a fraction of such a sample.

In certain embodiments, the method comprises determining the capacity of phase separation by detecting and/or measuring the presence, localization and/or morphology of a transcriptional condensate in a sample comprising said TF, particularly by an optical method, including but not limited to high-resolution imaging, confocal imaging, and stochastic optical reconstruction microscopy.

In further embodiments, the method comprises determining the composition of a transcriptional condensate comprising said TF, particularly by determining the presence and/or amount of at least one transcriptional cofactor. In some embodiments, the transcriptional co-activator is a compound which facilitates recruitment of the transcription apparatus including RNA Polymerase II. In some embodiments, the transcriptional co-activator has an enzymatic activity, including but not limited to kinase activity and/or acetyl-transferase activity, or has histone-binding activity. For example, the cofactor may be selected from Mediator or a sub-unit thereof, or BRD4.

The method of the present invention is suitable for detecting a genetic disease in a subject, particularly in a human subject, wherein the capacity of a Cluster 1 TF from said subject for phase separation is determined and/or the capacity of a Cluster 1 TF from said subject for forming a transcriptional condensate is determined. An altered capacity, particularly an altered capacity associated with an enhanced hydrophobicity versus the wild-type is indicative for a genetic disorder.

In certain embodiments, the genetic disorder may be selected from polysyndactyly, X-linked retardation, congenital ventral hypoventilation, hand-foot-genital syndrome, cleidocranial dysplasia, blepharophimosis, holoprosencephaly cephalic disorder, West syndrome, spinocerebrellar ataxia, spinal and bulbar muscular atrophy.

Further, the method of the present invention is suitable for screening a test compound whether it shows an effect on the capacity for phase separation and/or for forming a transcriptional condensate, and thus would be suitable for modulating a dysfunction of the TF and/or the transcriptional conjugate. For this purpose, the effect of the test compound on the capacity for phase separation and/or for forming a transcriptional condensate may be determined in the presence of a TF comprising an altered capacity versus a wild-type TF, particularly in the presence of a TF comprising a disease-associated mutation.

In certain embodiments, the test compound is a low-molecular weight compound, e.g. having a molecular weight of about 1000 Da or less, or even about 500 Da or less. In certain embodiments, the test compound is an amphiphilic compound comprising (i) a hydrophobic component, particularly a hydrophobic component comprising a hetero-aromatic nitrogen-containing ring, e.g. a purine or pyrimidine ring, and (ii) a hydrophilic component, e.g. comprising a sugar moiety and/or a polyvalent acid moiety, and wherein said test compound is more particularly a nucleoside phosphate, even more particularly a nucleoside triphosphate such as ATP.

A further aspect of the present invention relates to the therapeutic use of a modulator of the capacity of TF for phase separation and/or for forming a transcriptional condensate. Such a modulator can be used for preventing and/or treating a disorder associated with, caused by and/or accompanied with a dysfunction of a transcriptional condensate comprising at least one Cluster 1 mammalian TF. In certain embodiments, the modulator is used for preventing and/or treating a genetic disorder associated with, caused by and/or accompanied with a mutation in a Cluster 1 mammalian TF, e.g. a Cluster 1 human TF as described above. In certain embodiments, the genetic disorder may be selected from may be selected from polysyndactyly, X-linked retardation, congenital ventral hypoventilation, hand-foot-genital syndrome, cleidocranial dysplasia, blepharophimosis, holoprosencephaly cephalic disorder, West syndrome, spinocerebrellar ataxia, spinal and bulbar muscular atrophy.

In certain embodiments, the modulator is a low-molecular weight compound, e.g. having a molecular weight of about 1000 Da or less, or even about 500 Da or less. In certain embodiments, the test compound is an amphiphilic compound comprising (i) a hydrophobic component, particularly a hydrophobic component comprising a hetero-aromatic nitrogen-containing ring, e.g. a purine or pyrimidine ring, and (ii) a hydrophilic component, e.g. comprising a sugar moiety and/or a polyvalent acid moiety, and wherein said test compound is more particularly a nucleoside phosphate, even more particularly a nucleoside triphosphate such as ATP.

In some embodiments of the test compound or the modulator, the hydrophobic component (i) contains a moiety having a conjugated electron system, comprising at least 2 conjugated double bonds, e.g. C=C bonds, but also heteroatom-containing double bonds. For example, the moiety may comprise one or more aromatic ring systems, e.g. a phenyl or naphthyl ring and/or one or more hetero-aromatic ring systems, including, but not limited to a nitrogen-containing hetero-aromatic ring systems. In some embodiments, the hydrophobic compound comprises a hetero-aromatic nitrogen-containing ring, e.g. a purine or pyrimidine ring such as adenine, guanine, cytidine, thymine or uracil.

In some embodiments of the test compound or the modulator, the hydrophilic component (ii) comprises a polyvalent acid moiety including any acid salt and/or a sugar moiety. For example, the polyvalent acid is a phosphoric acid, including any phosphate, e.g. a mono-, di- or triphosphoric acid including a mono-, di- or triphosphate. In some embodiments, the hydrophilic component further comprises a sugar moiety and a phosphoric acid moiety.

Thus, in specific embodiments, the test compound or the modulator comprises (i) a hetero-aromatic nitrogen-containing ring and (ii) a sugar moiety and/or phosphoric acid moiety, and wherein said test compound or modulator is more particularly a nucleoside phosphate, even more particularly a nucleoside triphosphate such as ATP.

The modulator is administered in a therapeutically effective dose to a subject in need thereof, particularly to a human subject. A therapeutically effective dose may be determined by skilled practitioner based on the type and the severity of the disorder to be treated. The modulator may be administered by suitable local or systemical administration routes, e.g. by oral, nasal, transdermal, transmucosal such as buccal, vaginal, or rectal, ocular, or parenteral such as intravenous, intramuscular or subcutaneous administration. Administration of the modulator may result in an alteration of the condensation capacity of the TF comprising a disease-associated mutation, an alteration of the composition of a condensate comprising said TF and/or an alteration of the transcriptional activity of said TF or a condensate comprising said TF.

Still a further aspect of the present invention is an in vitro use of an amphiphilic molecule as described above for modulating the capacity of a Cluster 1 mammalian TF for phase separation and/or for forming a transcriptional condensate.

Further, the invention is explained in more detail by the following figures and examples.

### LEGENDS OF FIGURES

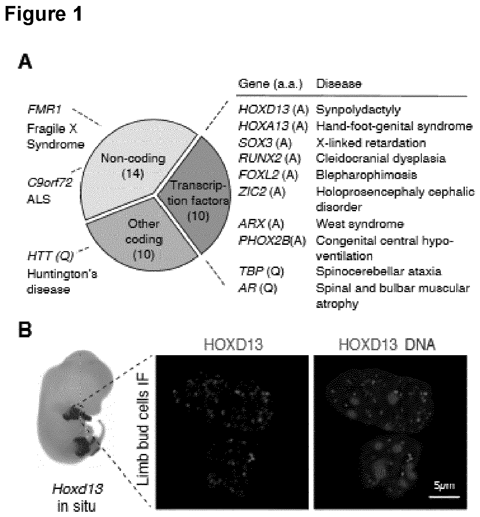

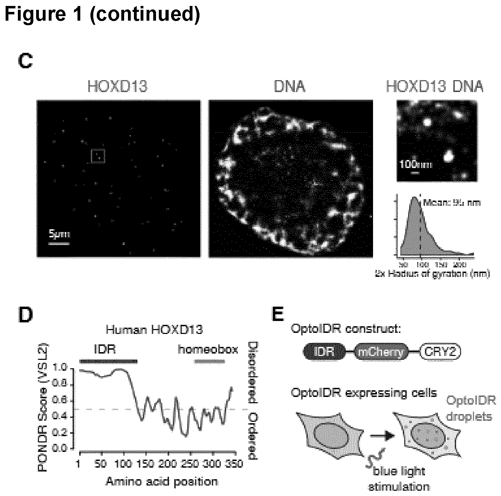

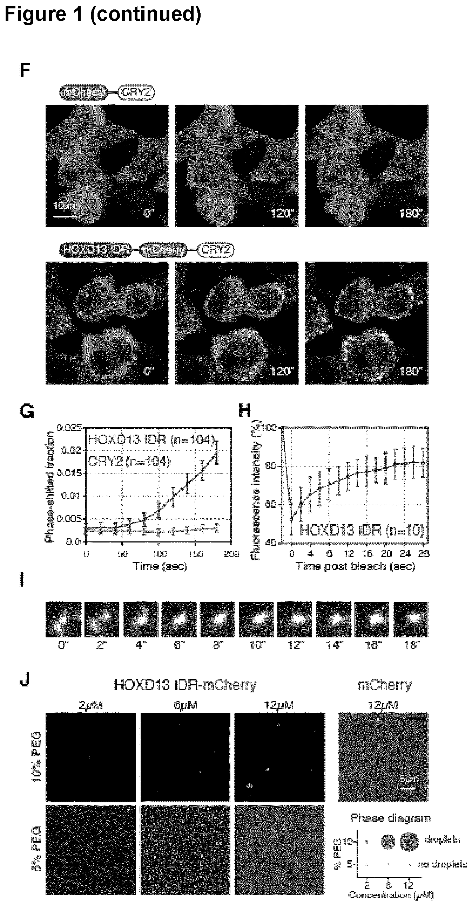

**Figure 1****. The HOXD13 IDR drives phase separation**
   **(A)** Disease-associated repeat expansions in humans. (a.a: amino acid)
   **(B)** (left) *Hoxd13* whole mount in situ hybridization in an E12.5 mouse embryo. (right) HOXD13 Immunofluorescence (IF) in E12.5 mouse limb bud cells.
   **(C)** Stochastic optical reconstruction microscopy (STORM) images of E12.5 mouse limb bud cells. The zoomed-in area on the right is highlighted with a red box on the left.
   **(D)** Graph plotting intrinsic disorder for human HOXD13. The IDR cloned for subsequent experiments is highlighted with a purple bar.
   **(E)** Scheme of the optoDroplet assay. The optoIDR construct consists of the HOXD13 IDR fused to mCherry and the *A. thaliana* CRY2 PHR domain.
   **(F)** Representative images of live HEK-293T cells expressing mCherry-CRY2 (top) and HOXD13 IDR-mCherry-CRY2 (bottom) fusion proteins. Cells were stimulated with 488nm laser every 20s for 3 minutes.
   **(G)** Quantification of the fraction of the cytoplasmic area occupied by HOXD13 IDR-mCherry-CRY2 and mCherry-CRY2 droplets in HEK-293T cells over time. Data displayed as mean+/-SEM.
   **(H)** Fluorescence intensity of HOXD13 IDR-mCherry-CRY2 droplets before, during and after photobleaching. Data displayed as mean+/-SD.
   **(I)** Time lapse images of a droplet fusion event in HEK-293T cells expressing HOXD13 IDR- mCherry-CRY2 fusion protein.
   **(J)** (left) Representative images of droplet formation by purified HOXD13-mCherry and mCherry at the indicated concentrations. (right) Phase diagram of HOXD13-mCherry in the presence of different concentrations of PEG-8000. The size of the circles is proportional to the size of droplets detected in the respective buffer conditions.

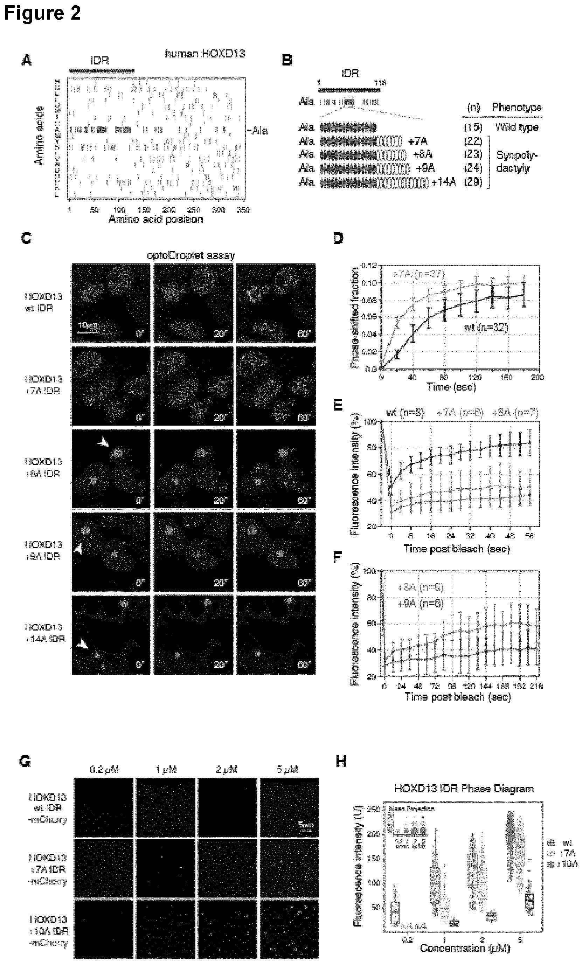

**Figure 2****. Synpolydactyly-associated repeat expansions enhance HOXD13 IDR phase separation**
   **(A)** Amino acid composition of human HOXD13. Ticks represent amino acids indicated on the y-axis at the positions indicated on the x-axis. The IDR cloned for subsequent experiments is highlighted with a purple bar.
   **(B)** Alanine residues (As) within the HOXD13 IDR sequence are indicated as red ticks. The central alanine repeat consists of 15 As in the wild-type (wt) protein.
   **(C)** Representative images of live HEK-293T nuclei expressing wt and repeat-expanded HOXD13 IDR-mCherry-CRY2 fusion proteins. Cells were stimulated with 488 nm laser every 20 s for 3 minutes. Arrowheads highlight spontaneously forming IDR condensates present without 488 nm laser stimulation.
   **(D)** Quantification of the fraction of the nuclear area occupied by HOXD13 wt IDR-mCherry-CRY2 and HOXD13 +7A IDR-mCherry-CRY2 droplets in HEK-293T cells over time. Data displayed as mean+/-SEM.
   **(E)** Fluorescence intensity of light-induced wt, +7A and +8A HOXD13 IDR droplets before, during and after photobleaching. Data displayed as mean+/-SD.
   **(F)** Fluorescence intensity of +8A and +9A spontaneously formed HOXD13 IDR condensates before, during and after photobleaching. Data displayed as mean+/-SD.
   **(G)** Representative images of droplet formation by purified HOXD13 IDR-mCherry fusion proteins in droplet formation buffer.
   **(H)** Phase diagram of HOXD13 IDR-mCherry fusion proteins. Every dot represents a detected droplet. The inset depicts the projected average size of the droplets as mean+/- SD (middle circle: mean, inner and outer circle: SD). n.d.: not detected.

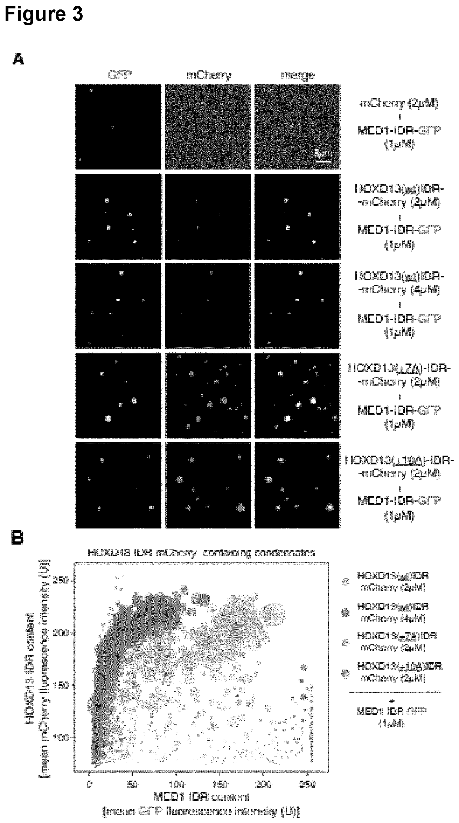

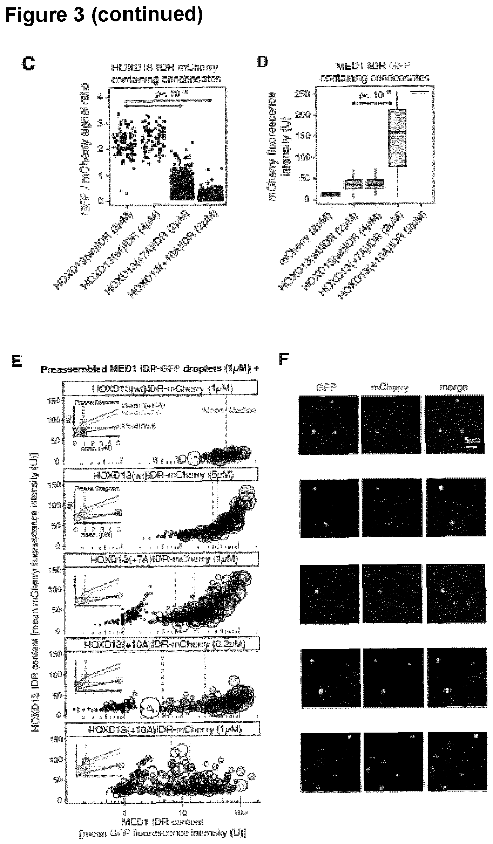

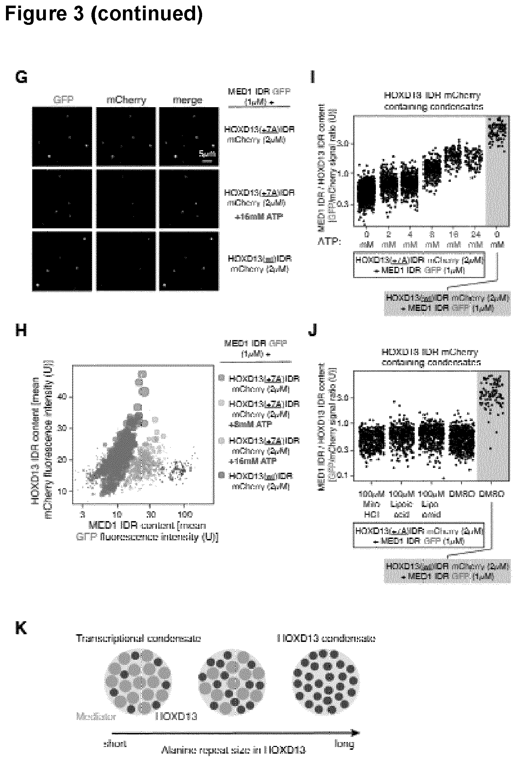

**Figure 3****. Synpolydactyly-associated repeat expansions alter the composition of Hoxd13-containing condensates in vitro**
   **(A)** Representative images of droplet formation by purified MED1-IDR-GFP and HOXD13 IDR-mCherry fusion proteins in droplet formation buffer.
   **(B)** Quantification of GFP and mCherry fluorescence intensity in HOXD13 IDR-mCherry containing droplets in the indicated MED1 IDR-GFP mixing experiments. Each dot represents one droplet, and the size of the dot is proportional to the size of the droplet.
   **(C)** Quantification of the ratio of GFP and mCherry fluorescence intensity in HOXD13 IDR-mCherry containing droplets in the indicated MED1 IDR-GFP mixing experiments. P value is from a Welch's t-test.
   **(D)** Quantification of mCherry fluorescence intensity in MED1 IDR-GFP containing droplets in the indicated MED1 IDR-GFP mixing experiments. P value is from a Welch's t-test.
   **(E)** Quantification of GFP and mCherry fluorescence intensity in HOXD13 IDR-mCherry containing droplets. Each dot represents one droplet. The size of the dot is proportional to the size of the droplet, and the color of the dot is scaled to the MED1 signal in the droplet. The insets show a simplified phase diagram of HOXD13 IDRs based on the data displayed in Figure 2H. x-axis is in log₁₀ scale.
   **(F)** Representative images of the mixtures in (E).
   **(G)** Representative images of droplets formed by purified MED1-IDR-GFP and HOXD13 IDR-mCherry fusion proteins.
   **(H)** Quantification of GFP and mCherry fluorescence intensity in HOXD13 IDR-mCherry containing droplets in the indicated MED1 IDR-GFP mixing experiments. Each dot represents one droplet, and the size of the dot is proportional to the size of the droplet. x-axis is in log₁₀ scale.
   **(I-J)** Quantification of the ratio of GFP and mCherry fluorescence in HOXD13 IDR-mCherry containing droplets in the indicated mixing experiments. In (I), the y-axis is in log₁₀ scale.
   **(K)** Condensate unblending model of the impact of HOXD13 alanine repeat expansions.

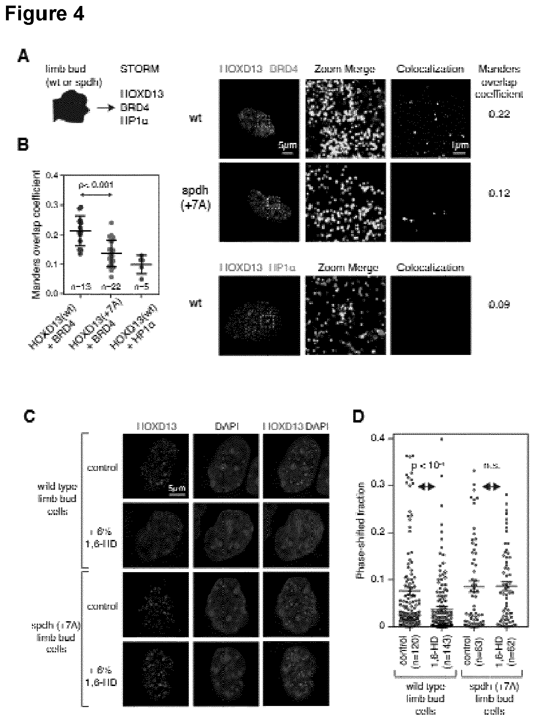

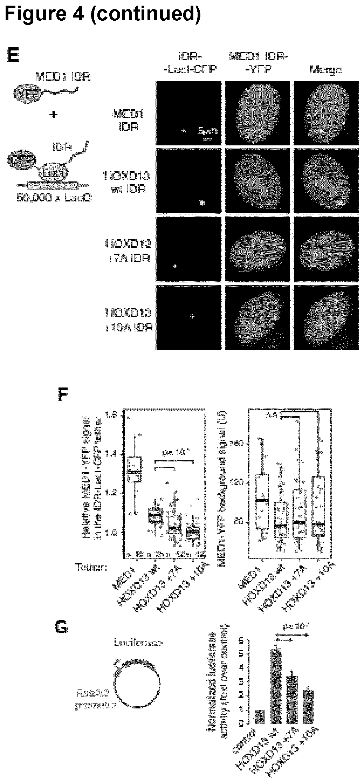

**Figure 4****. Altered composition and properties of repeat-expanded HOXD13-condensates in vivo**
   **(A)** (*left*) Experimental scheme (*right*) Stochastic optical reconstruction microscopy (STORM) images of wt and spdh E12.5 mouse limb bud cell nuclei. The zoomed-in area on the right is highlighted with a white box in the middle.
   **(B)** Manders overlap coefficients of the STORM co-localizations. P value is from an unpaired t-test.
   **(C)** (*left*) Experimental scheme (*right*) Representative images of wild type and spdh mouse limb bud cells with or without treatment with 6% 1,6-hexanediol for 1min.
   **(D)** Quantification of signal within HOXD13 puncta in mouse limb bud cells [displayed in (C)] with or without treatment with 6% 1,6-hexanediol for 1min.
   **(E)** Fluorescence images of ectopically expressed Med1 IDR-YFP in U2OS cells co-transfected with the indicated HOXD13 IDR-Lacl-CFP fusion constructs.
   **(F)** Quantification of the relative MED1 IDR-YFP signal intensity in the HOXD13 IDR foci. P values are from a Dunnett's test.
   **(G)** Luciferase reporter assays of HOXD13 wt, +7A and +10A mutants co-expressed with a *Raldh2*-luciferase reporter construct.

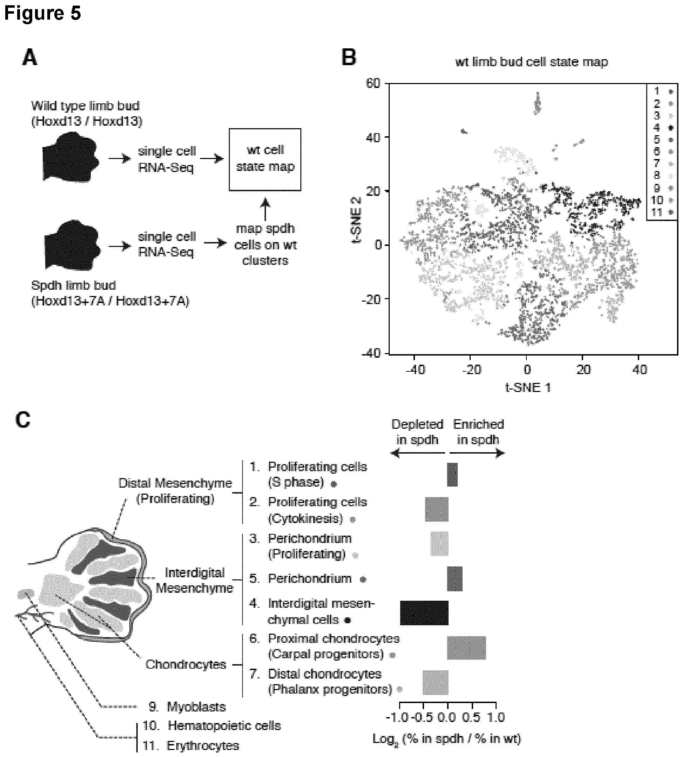

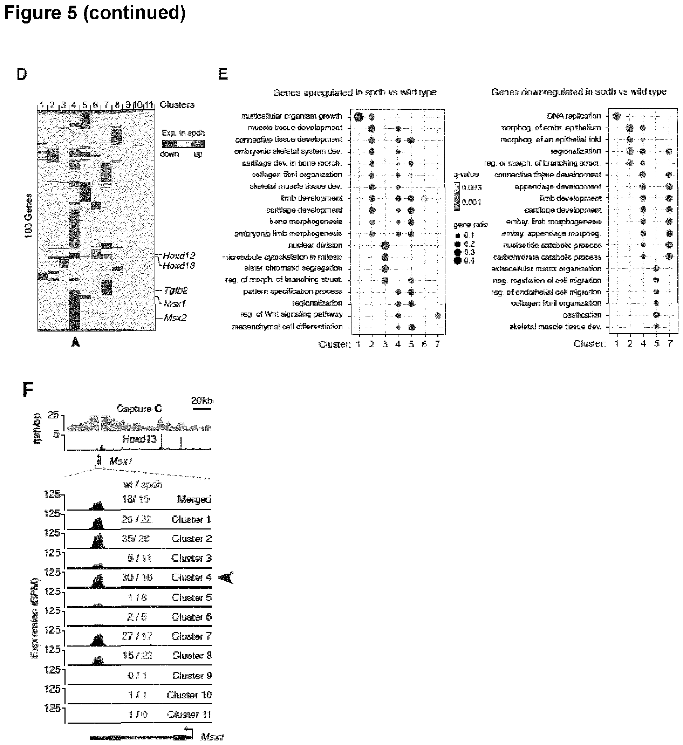

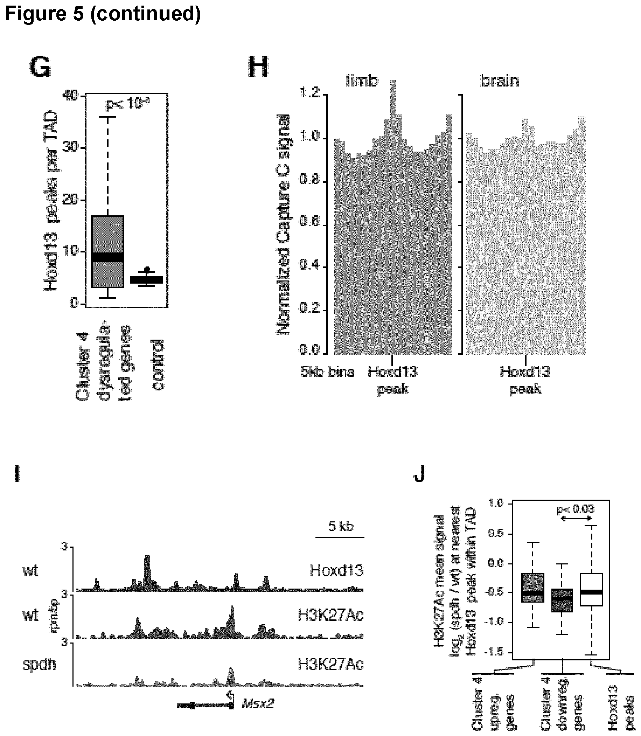

**Figure 5****. HOXD13 repeat expansion alters the transcriptional program of several cell types in a cell-specific manner**
   **(A)** Scheme of the scRNA-Seq experiment strategy.
   **(B)** Visualization of the wild-type scRNA-seq data using t-distributed Stochastic Neighbor Embedding (t-SNE).
   **(C)** Changes in cell type composition in spdh limb buds. Displayed are the relative changes in the proportions of cells that belong to the designated clusters (i.e. cell states) between wt and spdh limb buds.
   **(D)** Heatmap of differentially up- or downregulated genes in the spdh limb bud relative to wt within the 11 cell clusters. Arrowhead highlights the interdigital mesenchymal cells.
   **(E)** Gene Ontology (GO) term enrichment analysis of differentially up- or downregulated genes in the spdh limb bud relative to wt within individual cell clusters.
   **(F)** Profiles of Capture C, HOXD13 ChIP-Seq and scRNA-Seq data at the *Msx1* locus. The mean expression value in spdh (red) and wt cells (blue) within each cluster are also displayed. Arrowhead highlights the expression level in interdigital mesenchymal cells, where the expression difference is the most profound.
   **(G)** Number of HOXD13 peaks in topologically associating domains (TADs) that contain a gene dysregulated in Cluster 4. P value is from a Wilcoxon rank sum test.
   **(H)** Mean Capture C signal around HOXD13 peaks within topologically associating domains (TADs) that contain a gene dysregulated in Cluster 4.
   **(I)** ChIP-Seq binding profiles around the *Msx2* locus.
   **(J)** Quantification of the mean H3K27Ac signal at the nearest HOXD13 binding sites around the indicated genes within the same TAD. P value is from a Wilcoxon rank sum test.

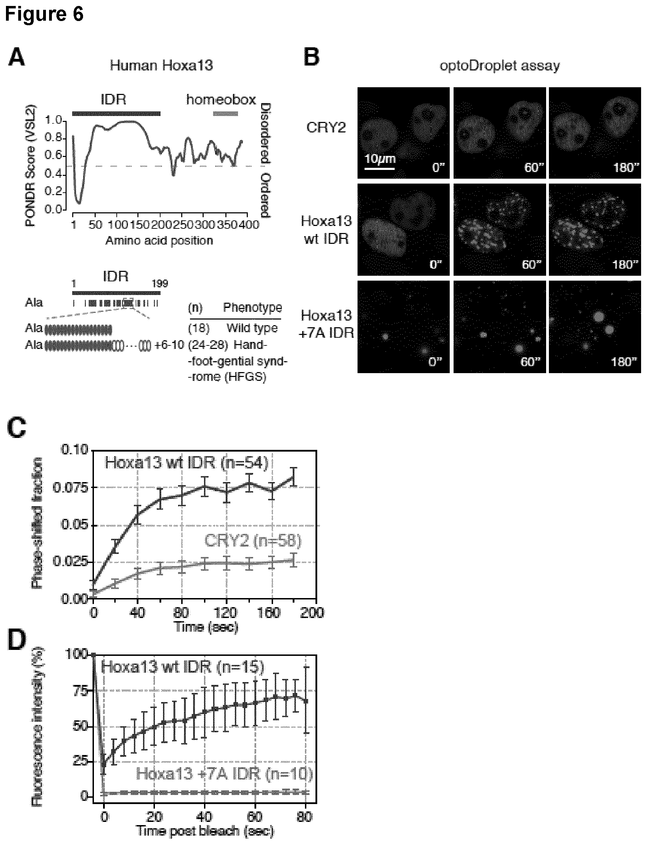

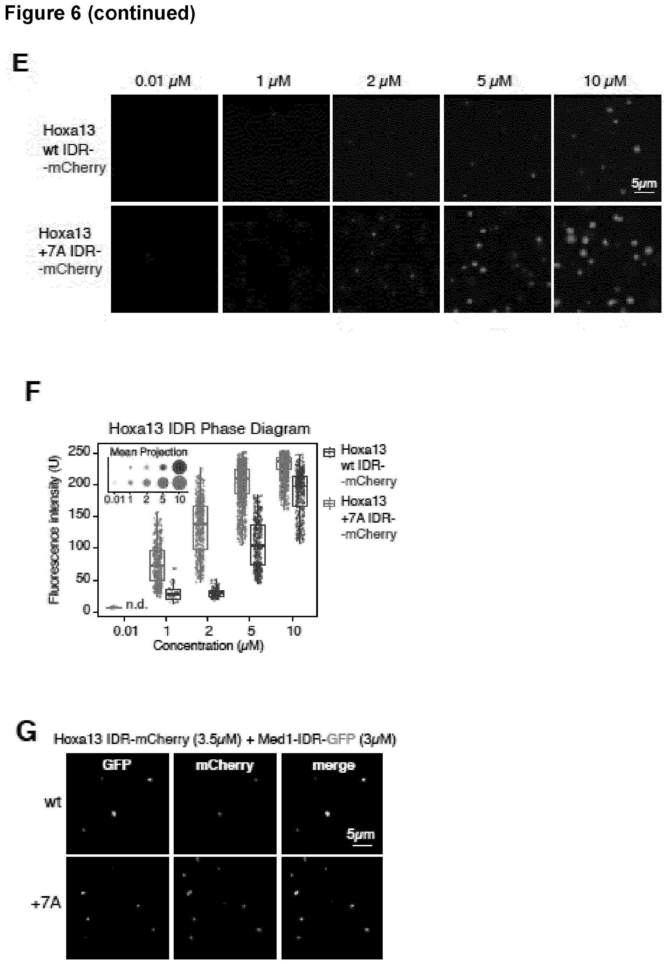

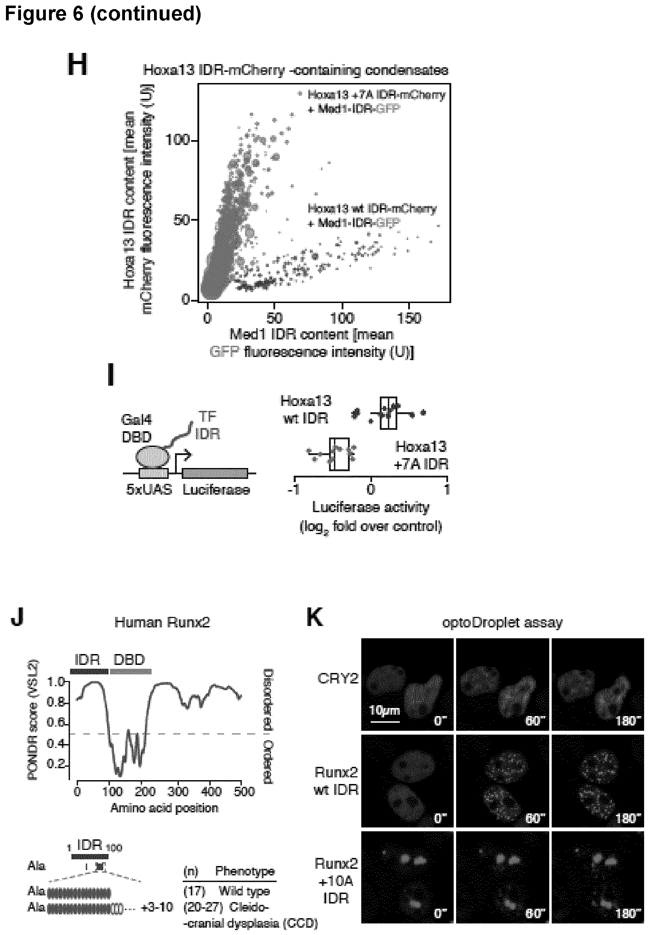

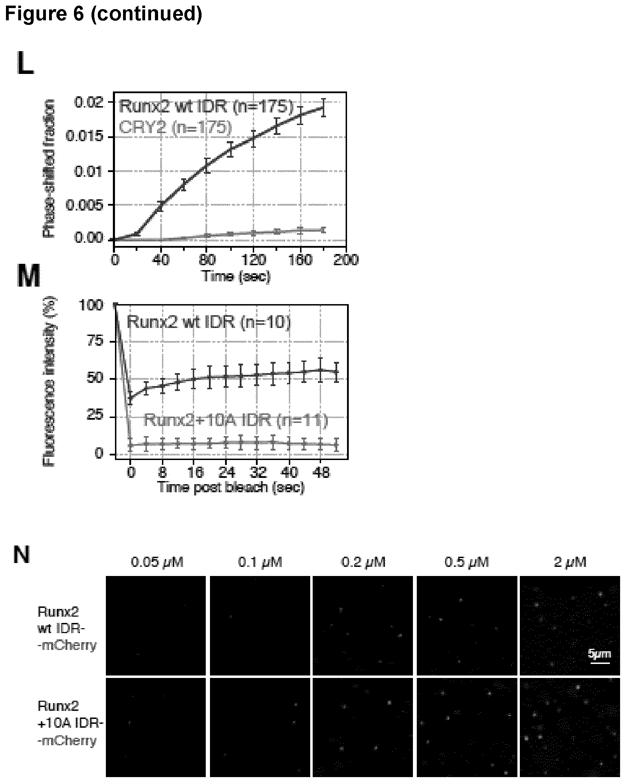

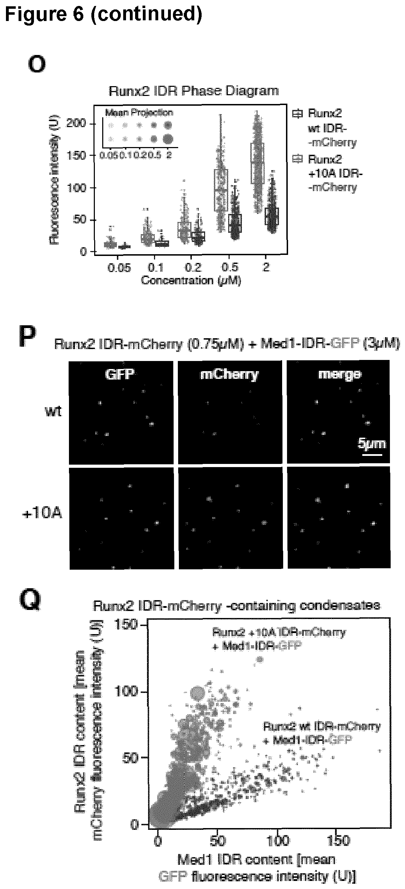

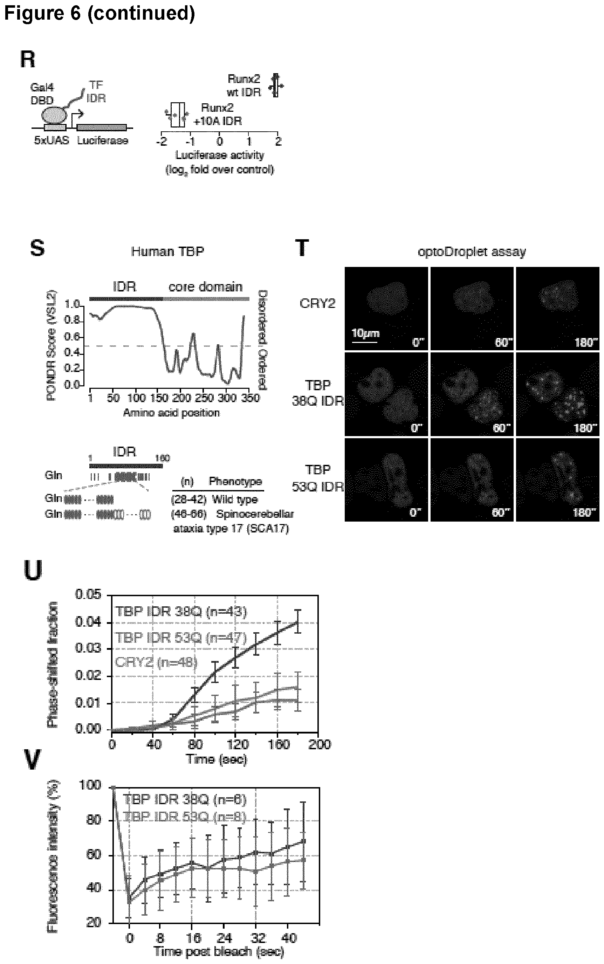

**Figure 6****. Disease-associated repeat expansions alter the phase separation capacity of other TF IDRs**
   **(A, J, S)** Graphs plotting intrinsic disorder for HOXA13, RUNX2 and TBP. The IDRs cloned for subsequent experiments are highlighted with a purple bar.
   **(B, K, T)** Representative images of HEK-293T nuclei expressing the indicated TF IDR-mCherry-CRY2 fusion proteins. Cells were stimulated with 488nm laser every 20s for 3 minutes.
   **(C, L, U)** Quantification of the fraction of the nuclear area occupied by droplets of the indicated TF IDR-mCherry-CRY2 fusion proteins in HEK-293T nuclei over time. Data displayed as mean+/-SEM.
   **(D, M, V)** Fluorescence intensity of droplets of the indicated TF IDR-mCherry-CRY2 fusion proteins before, during and after photobleaching. For the HOXA13 +7A IDR and the RUNX2 +10A IDR the spontaneously formed droplets were bleached, for all other fusion proteins the light-induced droplets were bleached. Data displayed as mean+/-SD.
   **(E, N)** Representative images of droplet formation by purified TF IDR-mCherry fusion proteins in droplet formation buffer.
   **(F, O)** Phase diagram of TF IDR-mCherry fusion proteins. Every dot represents a detected droplet. The inset depicts the projected average size of the droplets as mean+/- SD (middle circle: mean, inner and outer circle: SD). n.d.: not detected
   **(G, P)** Representative images of droplet formation by purified MED1-IDR-GFP and TF IDR-mCherry fusion proteins in droplet formation buffer with 10% PEG-8000.
   **(H-Q)** Quantification of GFP and mCherry fluorescence intensity in TF IDR-mCherry containing droplets in the indicated Med1 IDR-GFP mixing experiments. Each dot represents one droplet, and the size of the dot is proportional to the size of the droplet.
   **(I-R)** (left): GAL4 activation assay schematic. The luciferase reporter plasmid, and the expression vector for the GAL4 DBD-TF IDR fusion proteins were transfected into HEK-293T cells. (right): Luciferase reporter activity of the indicated TF IDRs fused to GAL4-DBD. *p* <10⁻³ for both wt/mutant comparisons (Dunnett's test).

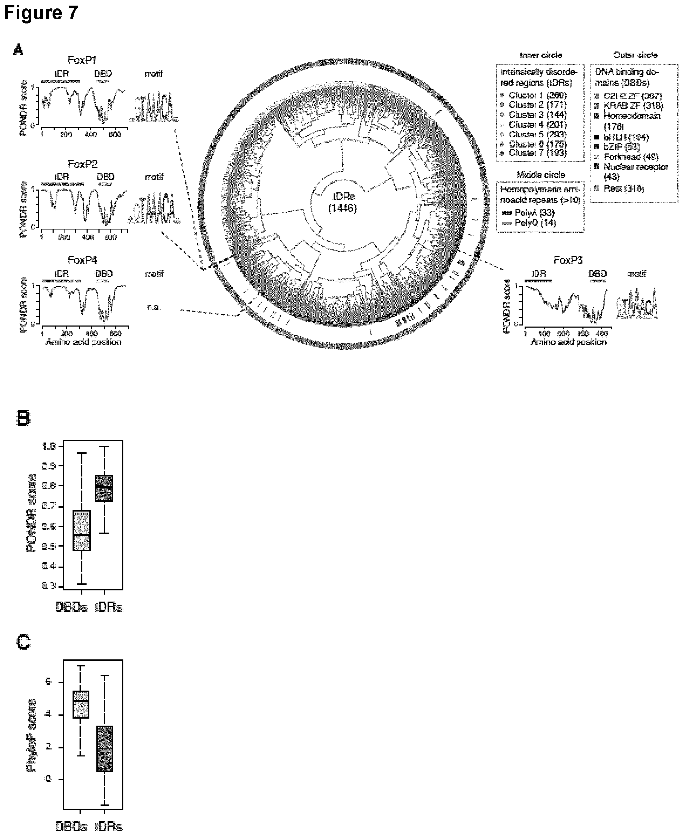

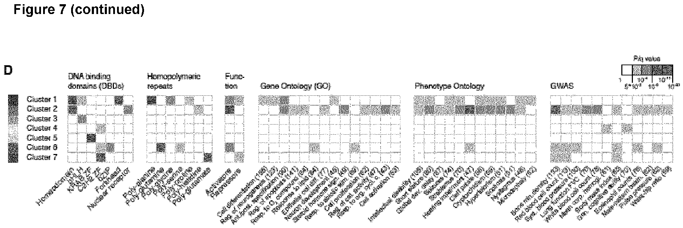

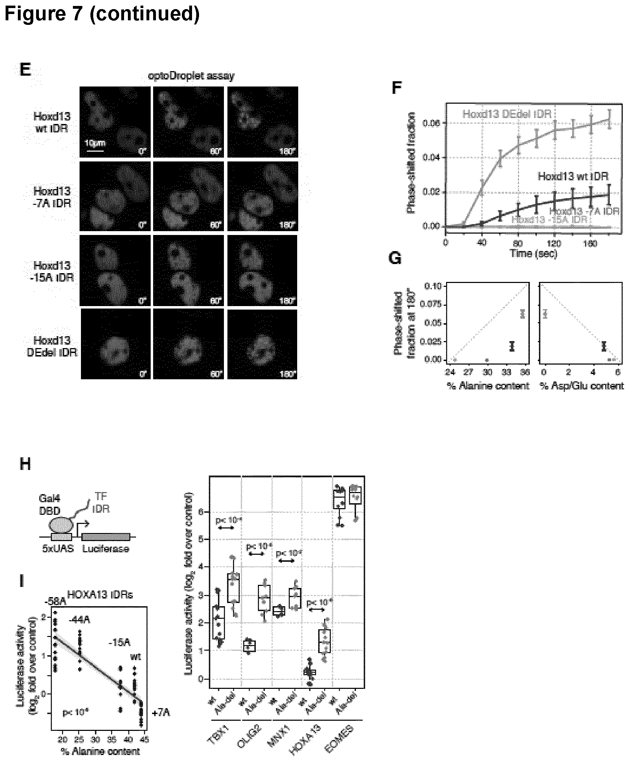

**Figure 7****. A catalog of human transcription factor IDRs**
   **(A)** Classification of human TF IDRs. The inner circle depicts the clusters of TF IDRs. The outer circle includes the annotation of the DBDs of the TFs whose IDRs were classified in the inner circle.
   **(B)** Boxplot of PONDR scores (disorder) of human TF DBDs and IDRs.
   **(C)** Boxplot of phyloP scores (conservation) of human TF DBDs and IDRs
   **(D)** Enrichment of TFs whose IDRs belong to the seven IDR clusters for the indicated sequence features, functional and phenotypic categories. Red box highlights significant enrichment (*q*<0.05).
   **(E)** Representative images of HEK-293T cells expressing the indicated HOXD13 IDR-mCherry-CRY2 fusion proteins. Cells were stimulated with 488 nm laser every 20 s for 3 minutes.
   **(F)** Quantification of the fraction of the nuclear area occupied by HOXD13 IDR-mCherry-CRY2 droplets in HEK-293T cells over time. Data displayed as mean+/-SEM.
   **(G)** Plot of the nuclear area occupied by HOXD13 IDR-mCherry-CRY2 droplets versus the Alanine content and Asp/Glu content of the IDR constructs. Data displayed as mean+/-SEM.
   **(H)** (left): GAL4 activation assay schematic. The luciferase reporter plasmid, and the expression vector for the GAL4 DBD-TF IDR fusion proteins were transfected into HEK-293T cells. (right): Luciferase reporter activity of the indicated TF IDRs fused to GAL4-DBD. P values are from a Dunnett's test.
   **(I)** Normalized luciferase activity of the indicated HOXA13 IDRs fused to GAL4 DBD. The blue line is a linear regression line, and the grey zones denote the 95% conference interval. P value is from a t-test.

### Examples

### 1. EXPERIMENTAL MODEL AND SUBJECT DETAILS

### Cell culture

HEK-293T, Kelly, SH-SY5Y, U2OS-2-6-3, and Cos7 cells were cultured in DMEM with GlutaMAX (ThermoFisher Scientific, 10566-016) supplemented with 10% FBS (Sigma Aldrich, F4135), and 100 U/mL penicillin-streptomycin (Gibco, 15140), at 37°C with 5% CO2 in a humidified incubator.

### Mouse husbandry

All animal procedures were conducted as approved by the local authorities (LAGeSo Berlin) under the license number #G0368/08.

### 2. METHOD DETAILS

### Construct generation

All optoIDR constructs used in this study were derived from the mCherry-Cry2WT plasmid (Addgene, 101221) described in (Shin et al., 2017). To generate mCherry-Cry2WT-NLS, a SV40 NLS primer pair (Sigma) was annealed and ligated into the mCherry-Cry2 plasmid. To generate optoIDR constructs, sequences containing the IDRs for Hoxd13wt, Hoxd13-7A, Hoxd13-15A, Hoxd13 DEdel, Hoxa13wt, Hoxa13, Hoxa13 +7A, TBP 38Q, TBP 53Q, Runx2, Runx2+10A were ordered as synthetic DNA from commercial vendors (Genewiz and IDT). IDR sequences were flanked by appropriate restriction sites for cloning. The IDR fragments were then ligated into mCherry-Cry2WT or mCherry-Cry2WT-NLS. For insertion of the alanine expansions +7A, +8A, +9A, and +14A into the Hoxd13-mCherry-Cry2- NLS construct, expansion oligonucleotides encoding respective alanine expansions were purchased (Sigma) and then inserted into the alanine stretch of Hoxd13-mCherry-Cry2-NLS using Gibson assembly, to generate Hoxd13(+7A)-mCherry-Cry2-NLS, Hoxd13(+8A)-mCherry-Cry2-NLS, Hoxd13(+9A)-Cry2-NLS, Hoxd13(+14A)-Cry2-NLS. All constructs were sequence verified. The nucleotide sequences of the constructs are shown in the attached sequence listing.
*SV40 NLS forward primer* (SEQ ID NO 1)
*SV40 NLS reverse primer* (SEQ ID NO 2)
*+7A oligonucleotide for Gibson assembly* (SEQ ID NO 3)
*+8A oligonucleotide for Gibson assembly* (SEQ ID NO 4)
*+9A oligonucleotide for Gibson assembly* (SEQ ID NO 5)
*+14A oligonucleotide for Gibson assembly* (SEQ ID NO 6)
*Hoxd13 wt gene fragment* (SEQ ID NO 7)
*Hoxa13 wt gene fragment* (SEQ ID NO 8)
*Hoxa13* +7A *gene fragment* (SEQ ID NO 9)
*TBP 38Q gene fragment* (SEQ ID NO 10)
*TBP 53Q gene fragment* (SEQ ID NO 11)
*Runx2 wt gene fragment* (SEQ ID NO 12)
*Runx2* +*10A gene fragment* (SEQ ID NO 13)
*Multiple cloning site gene fragment* (SEQ ID NO 14)

For the generation of IDR-YFP constructs, a YFP fragment was PCR amplified from an mCitrine vector and cloned into the mCherry-Cry2WT-NLS plasmid, to generate YFP-NLS. Hoxd13-IDR and Hoxd13(+7A)-IDRs were subcloned into YFP-NLS plasmid, to generate Hoxd13-IDR-YFP-NLS and Hoxd13(+7A)-IDRYFP-NLS. For the generation of Med1-IDR-YFP-NLS, the Med1-IDR was PCR amplified from a Med1 IDR expression vector (Boija et al., 2018; Sabari et al., 2018), and ligated into YFP-NLS.
*mEYFP forward primer* (SEQ ID NO 15)
*mEYFP reverse primer* (SEQ ID NO 16)
*Med1 forward primer* (SEQ ID NO 17)
*Med1 reverse primer* (SEQ ID NO 18)

For the generation of pET-IDR constructs, pET45b(+), mCherry was subcloned into pET45b(+) (Sigma Cat No. 71327), to generate pET-mCherry. IDRs were then PCR amplified from OptoIDR vectors using Q5 polymerase (NEB M0494S), and cloned into pET-mCherry using the NEBuilder HiFi DNA Assembly master mix (NEB E2621L). This pipeline was used to generate pET45-Hoxd13(WT)-IDR, pET45-Hoxd13(+7A)-IDR, pET45-Hoxd13(+10A)-IDR, pET45-Runx2(WT)-IDR, pET45-Runx2(+10A)-IDR, pET45-Hoxa13(WT)-IDR, pET45-Hoxa13(+7A)-IDR, and pET45-TBP37Q-IDR.

In the Gal4-DBD luciferase system, GAL4 DBD-IDR fusion constructs were expressed from a backbone of GAL4-DBD expression vector [GI-GAL4-DBD (Addgene 42500)], from which GIGAL4 sequence was removed. GAL4-DBD was PCR amplified from pCAG-GAL4-DBD-GBP2 vector (Addgene 49439) while introducing a short multiple cloning site C-terminal to GAL4-DBD allowing IDR cloning. Synthetic DNA fragments for wt and alanine repeat-deletion IDRs of GSX2, HOXD11, TBX1, EOMES, BHLHE41, and MNX1 sequences were flanked by AsiSI and BsiWI sequences for cloning, and purchased from Genewiz. For HEY2 and OLIG2, wt IDR sequences were PCR amplified from human iPSC gDNA and alanine repeat-deletion sequences were ordered from Genewiz. For HOXA13 and HOXD13, IDR sequences were PCR amplified from previously described vectors, except HOXA13 -44A and -58A deletion sequences were ordered from Genewiz. The HOXA13 -15A construct lacks alanines 88-103. In case of RUNX2, sequences were cloned from optoIDR constructs with Spel and BsiWI restriction enzymes and inserted to GAL4-DBD vector with a longer multiple cloning site with Nhel and BsiWI restriction sites.
*GAL4-DBD fw primer* (SEQ ID NO 19)
*GAL4-DBD (short MCS) rev primer* (SEQ ID NO 20)
*GAL4-DBD (long MCS) rev primer* (SEQ ID NO 21)
*OLIG2 fw primer* (SEQ ID NO 22)
*OLIG2 rev primer* (SEQ ID NO 23)
*HEY2 fw primer* (SEQ ID NO 24)
*HEY2 rev primer* (SEQ ID NO 25)
GSX2 wt sequence (SEQ ID NO 26)
GSX2 Ala-del sequence (SEQ ID NO 27)
HOXD11 wt sequence (SEQ ID NO 28)
HOXD11 Ala-del sequence (SEQ ID NO 29)
TBX1 wt sequence (SEQ ID NO 30)
TBX1 Ala-del sequence (SEQ ID NO 31)
EOMES wt sequence (SEQ ID NO 32)
BHLHE41 wt sequence (SEQ ID NO 33)
BHLHE41 Ala-del sequence (SEQ ID NO 34)
HOXA13 -58A (Ala-del) sequence (SEQ ID NO 35)
HOXA13 -44A sequence (SEQ ID NO 36)
MNX1 wt sequence (SEQ ID NO 37)
MNX1 Ala-del sequence (SEQ ID NO 38)
HEY2 Ala-del sequence (SEQ ID NO 39)
OLIG2 Ala-del sequence (SEQ ID NO 40)

For LacO-LacI experiments (Figure 4E-F), a vector containing CFP-LacI followed by a multiple cloning site (MCS) was prepared from CFP-LacI-MED1-IDR (Zamudio et al., 2019) by removing MED1-IDR with BamHI + EcoRI digestion. Next, multiple cloning site was introduced with annealed, BamHI + EcoRI digested oligonucleotides (below), and AsiSI/BsiWI sites were used to subclone HOXD13 IDR sequences from previously described GAL4-DBD-IDR vectors.
*MCS fw primer* (SEQ ID NO 41)
*MCS rev primer* (SEQ ID NO 42)

### Protein disorder analysis

Intrinsically disordered regions (IDRs) in Hoxd13, TBP, Hoxa13, Runx2, and Foxp1-4 (Figure 1D, 6A, 6J, 6S, 7A) were predicted using the publicly available Predictor of Naturally Disordered Regions (PONDR) algorithm (VSL2) (Peng et al., 2006) as previously described (Boija et al., 2018; Sabari et al., 2018).

### Isolation of limb bud cells

Limb buds from E12.5 wild type and spdh homozygous embryos were micro-dissected individually and digested with Trypsin-EDTA 0.05% (Gibco) for 15 minutes at 37°C and gently dissociated by pipetting after 5, 10, and 15 minutes. Cells were mixed in cell culture media (DMEM, 10% FBS, 2mM L-Glutamine, 50 U/ml Penicillin/Streptomycin) and a single-cell suspension was obtained using a 40 µm cell strainer (Falcon). About 150,000 limb bud cells from each embryo were seeded in 1000 µl medium onto fibronectin-coated glass coverslips in 12 well plates. After 30-60 minutes, additional cell culture medium was added, and cells were grown for 24 hours. After 24 hours, the cells were rinsed twice with PBS and fixed 15 min at room temperature with 4 % PFA/PBS.

### Whole mount in situ hybridization (WISH)

WISH was performed as previously described (Kuss et al., 2009; Villavicencio-Lorini et al., 2010).

### Cell treatments

Transfection: For transient transfection and live cell imaging, HEK-293T cells were seeded onto chambered coverslips (Ibidi, 80826-90), and transiently transfected 20-24 h later using lipoD293 (Signagen, SL100668) or FuGENE HD (Promega) according to the manufacturer's protocol.

1,6-hexanediol treatment: Isolated limb bud cells attached to fibronectin coated glass coverslips were treated with 6% or 0% hexanediol (Sigma, 240117) in 1 mL cell culture media for 1 minute at 37°C. After treatment, cells were washed with 1 mL of PBS, and immediately fixed in 4% paraformaldehyde in PBS for 10 min. Fixed cells were washed and then stored at 4°C until processing for immunofluorescence and microscopy.

### Western blot

Cells were washed once with ice-cold PBS, and lysed in RIPA buffer (Thermo, 88900) supplemented with protease inhibitors (Thermo, 87786). Protein concentration was determined using bicinchoninic acid assay (Thermo, 23225), according to the manufacturer's protocol. Lysates containing equal protein amounts were then heated to 95°C for 10 minutes, and separated on 4-12% Tris-acetate gels (Invitrogen, NP0322BOX). Protein was transferred onto a nitrocellulose membrane. After transfer, the membrane was blocked. The membranes were incubated with 1:750 anti-Hoxd13 (abcam ab229234) and 1:3000 anti-HSP90 (BD Biosciences, 610419) antibody diluted in 5% non-fat milk in TBST overnight at 4°C with shaking. The next day, membranes were washed, incubated with fluorescent anti-mouse (IRDye 800CW Donkey anti-Mouse, Li-Cor P/N 925-32212) and anti-rabbit (IRDye 680LT Donkey anti-Rabbit, Li-Cor P/N 925-68023) secondary antibodies at 1:10000 dilution according to the manufacturer's protocol, and finally washed in the dark. Membranes were imaged on a LICOR Odyssey Clx imager.

### Immunofluorescence

Isolated limb bud cells, Kelly cells, and SH-SY5Y cells attached to coated glass coverslips were fixed in 4% paraformaldehyde (PFA) (Sigma-Aldrich, P6148) in PBS for 10 min and stored at 4°C in PBS or processed immediately. Cells were permeabilized in 0.1% Triton X-100 (Thermo Scientific, 85111) in PBS for 10 min at RT. Then, after washing and blocking, cells were incubated with the primary antibody (anti-Hoxd13 Invitrogen PA5-66661 1:250 dilution or anti-Hoxd13 abcam ab19866 1:150 dilution) in blocking solution at 4°C overnight. After washing, cells were incubated with the secondary antibody (donkey anti-Rabbit IgG Alexa Fluor 568 Invitrogen A10042 1:1000 dilution) in blocking solution in the dark. Nuclei were counterstained with 0.24 mg/mL 4',6-diamidino-2-phenylindole (DAPI) in PBS in the dark. Coverslips were mounted onto slides with Vectashield (Vector, H-1000) and sealed. Images were acquired at the confocal laser-scanning microscope (Zeiss LSM880, 63x oil objective, NA 1.4, 1 Airy Unit). Raw images (.czi files) were processed in FIJI. The abcam ab19866 antibody was used in Figure 1B; otherwise, the primary antibody is indicated in the respective figure panels.

### Stochastic optical reconstruction microscopy (STORM)

Isolated E12.5 limb bud cells attached to fibronectin coated glass coverslips were fixed in 4% paraformaldehyde (PFA) (Sigma-Aldrich, P6148). The fixed samples were washed, treated with permeabilization solution (PBS supplemented with 0.1% Triton X-100) and treated with blocking solution (permeabilization solution supplemented with 10% fetal bovine serum). After blocking, samples were incubated with primary anti-Hoxd13 antibody (Thermo, PA5-66661, 1:250 in blocking solution), BRD4 antibody (Clone A-7, Santa Cruz, sc-518021, 1:250 in blocking solution), or HP1α antibody (clone15.19s2, Millipore/Merck, 1:250 in blocking solution), and then washed. Stained and washed samples were then incubated with secondary antibody (goat anti-Rabbit IgG Alexa Fluor 647 Invitrogen A32733 / goat anti-Rabbit IgG Cy3 Invitrogen A21235, 1:1000 in blocking buffer for Hoxd13 primary and goat anti-Mouse IgG Alexa Fluor 647 Invitrogen A10520, 1:1000 in blocking buffer for BRD4/HP1α primary), after which samples were wahed. For imaging, samples were placed in a one-well magnetic chamber, covered in switching buffer consisting of 0.15 M 2-mercaptoethanol/0.2 M Tris, pH 8.0 with 4 % (w/v) glucose, 0.25 mg/ml glucose oxidase, 20 µg/ml catalase and 5 nM Sytox Orange (Thermo, S11368). Images were acquired with a Vutara 352 super resolution microscope (Bruker) equipped with a Hamamatsu ORCA Flash4.0 sCMOS for super resolution imaging and a 60x oil immersion TIRF objective with numerical aperture 1.49 (Olympus). Data were acquired with TIRF/HILOillumination at a laser-power density of 62.5 kW/cm² using a 532 and 639 nm laser.

### STORM image analysis

STORM images were analyzed using Vutara SRX software (Bruker) or the Picasso software package (Schnitzbauer et al., 2017).

For Hoxd13 localization and cluster analysis (Figure 1C), images were collected with a 50 ms acquisition time. Hoxd13 and Sytox Orange localizations were analyzed using the Picasso software package, as described in (Fabricius et al., 2018). The NeNa localization precision (Endesfelder et al., 2014) was 9 nm for Hoxd13 localizations.

For Co-IF STORM analysis (Figure 4A-B), 4000 images were collected with a 50 ms acquisition time for each cell (2000 images for each probe). Detected Hoxd13, BRD4 or HP1alpha particles were then localized using the Vutara SRX single molecule localization tool, and subsequently projected to create density maps (Figure 1C, 4A). Overlaps between localized probes was assessed by calculating the Mander's overlap coefficient between probes for each cell using the SRX visualization tool. Each cell was analyzed separately for overlap between probes, and means of the Mander's coefficient (N = 20-25 for Hoxd13 / BRD4 co-localizations, N = 5 for Hoxd13 / HP1alpha co-localizations) were compared using unpaired, two-tailed student's t-tests (Figure 4B).

### Live cell imaging

All live cell imaging experiments were performed on an LSM880 confocal microscope (Zeiss) equipped with an incubation chamber with a heated stage at 37°C. Images were acquired with either a Plan-Apochromat 40x0.95 Korr M27 or a 63x1.40 oil DIC objective. OptoDroplet assay: The optoIDR assay was adapted from (Shin et al., 2017). Briefly, approximately 20,000 cells were seeded per well on chambered coverslips one day before transfection. The following day, cells were transfected with 200 ng of the respective optoIDR construct. 48 hours post-transfection, culture medium was refreshed, and cells were imaged on a Zeiss LSM 880 confocal microscope. Droplet formation was induced with scans with the 488 nm laser every 20 seconds for the duration of imaging (Figure 1F-G, 2C-D, 6B-C, 6K-L, 6T-U, 7E-F). For image acquisition, mCherry fluorescence was stimulated at 561 nm laser every 20 seconds. The constructs used for the optoDroplet experiments described in Figure 1F-1I did not include the SV40 NLS sequence, and the fusion protein displayed cytoplasmic localization. All other data was generated using constructs that included the SV40 NLS.

For FRAP experiments of light induced droplets (Figure 1H, 2E, 6D, 6M, 6V), droplet formation was induced with continuous 488 nm light for 90 seconds. Droplets were then bleached with 561 nm light, and recovery was imaged every 4 seconds in the presence of simultaneous 488 nm light stimulation. For FRAP experiments of spontaneous IDR bodies of Hoxd13 (Figure 2F) bodies were bleached with 561 nm light and recovery was imaged every 12 seconds.

LacO-LacI assays (Figure 4E-F): tethering experiments were adapted from (Chong et al., 2018; Zamudio et al., 2019). Briefly, 20,000 U2OS cells were seeded on chambered coverslips one day before co-transfecting the cells with 100 ng of CFP-Lacl-HOXD13-IDR plasmid and 100 ng of Med1-IDR-YFP-NLS plasmid with FuGENE HD reagent (Promega). Imaging was performed on live cells 48-80 h after transfections.

### Optodroplet assay analysis

All data analysis on images was performed in Zen Blue 2.5 (Zeiss) Arivis Vision4D or ImageJ. For optoDroplet analysis in Zen Blue 2.5, nuclear and cytoplasmic mCherry signals were automatically thresholded and size filtered to define primary regions of interest. Within these primary regions, optoDroplets were detected using a second fixed pixel intensity threshold. For optoDroplet analysis in Arivis Vision4D, images were first converted to an appropriate file format as multiple scope files in Arivis SIS converter. Nuclear mCherry signals were automatically thresholded and size-filtered to define primary regions of interest. OptoDroplets were detected as secondary regions of interest. The pipeline was then used to analyze at least 50 - 200 cells per genotype (indicated by in each figure panel). The phase-shifted fraction was calculated as the total area of detected optoDroplets within a nucleus divided by the area of the corresponding cell's nucleus (Figure 2D, 6C, 6L, 6U, 7F). In Figure 1G, where non-nuclear IDR constructs were used, the phase-shifted fraction was calculated as the total area of detected optoDroplets within a cell divided by the area of the corresponding cell. Cells were filtered for expression level (outlier cells were removed). The ratios were averaged over all detected cells per the indicated optoIDR genotypes, and plotted over time.

For phase-shifted fraction comparison in the Hoxd13 deletion series in Figure 7E-F, 30 cells in the mCherry signal intensity range of 75 to 100 at initial timepoint were selected at random for each genotype using Arivis Vision4D processing as described above. The phase-shift score was averaged over the 30 cells per genotype, and plotted over time. IDR properties, namely the % residue composition was computed from the amino acid sequence using the ProtParam tool and plotted against the mean phase-shift score at 180" of green light stimulation per genotype (Figure 7G).

For FRAP analysis, mean pixel intensity of regions of interests were measured using ImageJ, and normalized to pre-bleaching intensity. Captured intensity traces were averaged over multiple replicates for each genotype (indicated as n in figure panels), and values were plotted against time.

### Confocal/Fluorescence image analysis

Hoxd13 puncta detected in fixed cell immunofluorescence (Figure 4C-D) were analyzed using a similar workflow described above. Nuclear regions were automatically detected in the Zen Blue (Zeiss) software package using the DAPI counterstain signal. Puncta were segmented on morphology, and then filtered based on fluorescence intensity and circularity. After adjusting parameters on 4-5 images, the established pipeline was used to analyze all images from cell-types and treatment conditions (7 images for untreated wild type limb bud, 7 images for 6% 1-6 HD treated wild type limb bud, 18 images for untreated spdh limb bud, 12 images 6% 1-6 HD treated spdh limb bud). Values for nuclear area, Hoxd13 puncta area, Hoxd13 puncta mean fluorescence intensity, and Hoxd13 puncta count were measured for 120 wild type cells, 143 1-6 HD treated wild type cells, 63 spdh cells, and 62 1-6 HD treated spdh cells.

### In vitro droplet assay analysis

For image analysis, droplets were first automatically thresholded in either the mEGFP or mCherry channel using Zen Blue (Version 3.0), and then filtered by size, pixel intensity, and pixel deviation. Mean fluorescence intensities, area, and diameter of these primary regions were measured on both channels. For quantification and data visualization (Figure 1J, 2H, 3B-E, 3H-J, 6F, 6H, 6O, 6Q), raw values for diameter, mean intensity, or area were plotted as indicated in figure legends using R-Studio and the Directlabels package.

### LacO-LacI tethering image analysis

For LacO-LacI image analysis (Figure 4E-F) primary regions of interest corresponding to IDRLacl-CFP tethers were detected based on a fixed intensity threshold on the cyan channel. Mean intensities of these primary regions were measured on both YFP and cyan channels. To quantify partitioning of Med1 IDR-YFP constructs, normalized signals of primary regions, in both channels, were plotted against the corresponding co-transfections, as indicated in Figure 4F. For quantification, tethers within cells of similar expression levels of both YFP and CFP constructs were used, as indicated in Figure 4F.

### Statistical analysis

Statistical analyses of microscopy image quantifications were carried out in Graphpad Prism and R. Overlap analysis of super-resolution images was carried out using Vutara SRX analysis software.

### Protein purification and in vitro droplet formation assays

For protein expression, plasmids were transformed into BL21(DE3) (NEB M0491S) cells, and grown in automatic-induction medium (AIM), as described in (Studier, 2005). For protein purification from the cultivated cells, pellets were resuspended in 30 mL of Buffer A (50 mM Tris pH 7.5, 300 mM NaCl) supplemented with 8 M Urea and cOmplete protease inhibitors (Sigma, 11697498001). This denatured suspension was sonicated and clarified by centrifugation. Supernatants containing fusion proteins were loaded onto a His GraviTrap column (GE HealthCare, 11003399) pre-equilibrated in Buffer A supplemented with 8 M Urea. The loaded column was washed with 6% Buffer B (50 mM Tris pH 7.5, 300 mM NaCl, 500 mM imidazole) in Buffer A supplemented with 8 M Urea, and 6% Buffer B. Proteins were eluted in 50% Buffer B, immediately diluted 1:2 in storage buffer (50 mM Tris pH 7.5, 125 mM NaCl, 1 mM DTT, 10% Glycerol), and then concentrated. The resulting fraction was then diluted 1:1000 in storage buffer, re-concentrated, and then stored at -20°C. All fusion proteins were purified in the same manner.

For in vitro droplet formation, recombinant mCherry or mEGFP fusion proteins were measured for concentration, and then diluted or mixed to suitable final concentrations in storage buffer. For microscopy, these solutions were further diluted 1:2 in either 20% or 10% PEG-8000 in deionized water (w/v). 10 µL of this suspension was pipetted onto chambered coverslips, and immediately imaged using a LSM880 confocal microscope equipped with a 63x1.40 oil DIC objective and AiryScan detector. All images were acquired from within the solution interface, and performed before droplets settled onto the bottom of the coverslip, as described in (Sabari et al., 2018). For droplet assays using preformed GFP-Med1 condensates (Figure 3E-F), GFPMed1 droplets were allowed to form for 30 minutes at room temperature in the presence of 10% PEG-8000, before proceeding with co-condensation assays. For compound treatments of cocondensates (Figure 3G-J), small molecules [ATP (Jena Bioscience, NU-1010), Mitoxantrone dihydrochloride (Sigma M6545), (±)-α-lipoic acid (Sigma 62320), and lipoamide, (Medchemexpress HY-B1142)] were directly diluted into droplet mixtures with or without vehicle (DMSO, Sigma D2650) to the desired final concentrations.

### Luciferase reporter assays

The murine proximal enhancer of *Aldh1a2* (Kuss et al., 2009) was cloned into the pGL2-enhancer luciferase reporter vector and co-transfected into Cos7 cells with pcDNA3.1 (wild type or mutant Hoxd13) expression plasmids and pRL-CMV (Promega) using FuGENE HD (Promega) according to the manufacturer's instructions. 24 hours post transfection, reporter activity in 2.5 µl of cell lysate was measured using the Dual-Glo Luciferase Assay System (Promega) according to the manufacturer's instructions (Figure 4G).

Measurements were performed similarly in Gal4-DBD luciferase assays (Figure 6I, 6R, 7H-I), except GAL4UAS-Luciferase reporter [Addgene 64125, described in (Nihongaki et al., 2015)] was co-transfected with GAL4-IDR fusion constructs into HEK-293T cells, and with pRLCMV, and 10 µl of cell lysate was used in measurements.

### H3K27Ac ChIP-seq with S2 spike-in control (ChIP-RX)

Isolated E12.5 limb bud cells (hand plate) from 2 wild-type, 2 homozygous spdh, and 2 heterozygous spdh mice (approximately 3.5 million cells each) across 2 separate het x het mouse crosses were fixed in 1% formaldehyde on ice and quenched with 2.5 M glycine. Each pool was spiked in with 1 million fixed fly S2 cells (Orlando et al., 2014), after which cell suspensions were lysed in lysis buffer (50 mM HEPES, 140 mM NaCl, 1 mM EDTA, 10% Glycerin, 0.5% NP-40, 0.25 Triton X-100, pH 7.5 supplemented with Protease inhibitors and Na-Butyrate). After shearing, 10-15 µg chromatin was incubated with 4 µL of H3K27Ac antibody (Diagenode C15410174) in a total of 1.2 mL of buffer (10 mM Tris-HCl, 100 mM NaCl, 1 mM EDTA, 0.5 mM EGTA, 0.1% Na-DOC, 0.5% NLaroylsarcosine, pH 8.0 supplemented with Protease inhibitors and Na-Butyrate) overnight at 4°C. After incubation, 30 µL of Protein G beads were added to the chromatin and antibody suspensions, and allowed to incubate overnight at 4°C. Samples were then washed with 1 mL of RIPA buffer (to 50 mM HEPES-KOH, 1 mM EDTA, 1% NP-40, 0.7% Na-DOC, 500 mM LiCI, pH 7.55 supplemented with Protease inhibitors and Na-Butyrate). Beads were then washed with TE buffer (supplemented with protease inhibitors and Na-butyrate) and then centrifuged down to remove TE buffer. Chromatin was then eluted using 210 µL of elution buffer (50 mM Tris-HCl, 10 mM EDTA, 1% SDS, pH 8.0) and heated to 65°C. Eluates were then treated with 5 µL of Proteinase K overnight at 65°C. The next day, 4 µL of RNAse A was added to the samples, vortexed, spun down and incubated at 37°C for 30 minutes. Chromatin was then extracted using phenolchloroform, and precipitated using 70% ice cold EtOH, with centrifugation at max speed for 10 minutes. Supernatant was removed, and the resulting pellet was dissolved in deionized water. Total yield was assessed by Qubit, and then sent for sequencing. Respective ChIP samples and input controls were paired-end sequenced using Illumina high-throughput sequencing, to a depth of around 25 million reads.

### Cluster identification

To identify cell populations in the wild type scRNA-Seq data the following Seurat functions were used: FindVariableGenes, RunPCA, RunTSNE and FindClusters (Butler et al., 2018). Clusters were identified by first building a shared nearest neighbor graph and then running the Louvain algorithm on it (Blondel et al., 2008).

### Assignment of cell types to clusters

The marker genes in the 11 clusters of the wild type reference map were identified by running the FindAllMarkers functions of the Seurat package (Butler et al., 2018). The clusters were then assigned to cell types using a GO term enrichment analysis, inspection of localized gene expression data from whole mount in situ hybridization [e.g. using the Gene expression Database (GXD)], and literature data on marker genes of cell types in the developing limb..

Cluster 1: Proliferating cells (S phase) of the distal proliferating mesenchyme; Cluster 2: Proliferating cells (Cytokinesis) of the distal proliferating mesenchyme; Cluster 3: Perichondrium (Proliferating mesenchyme); Cluster 4: Interdigital mesenchyme; Cluster 5: Perichondrium; Cluster 6: Proximal chondrocytes (carpal progenitors); Cluster 7: Distal chondrocytes (phalanx progenitors); Cluster 8: Proliferating cells; Cluster 9: Myoblasts; Cluster 10: Hematopoietic cells; Cluster 11. Erythrocytes.

### Quantification of cell state proportions

Spdh cells were assigned to the identified 11 clusters of the wild type reference cell state map using the nearest neighbor method. Spdh cells were assigned to the wild type cluster that had the most similar mean expression profile. For each cluster and condition we normalized the cell counts by dividing by the total number of wild type or spdh cells used. Last, we determined the log2 ratio of these normalized cell state percentages in the spdh and wild type samples per cluster (Figure 5C).

### Differential expression analysis

Differential expression analysis was performed within clusters between wild type and assigned spdh cells by using the FindMarkers function of the Seurat package. To display differentially expressed genes in Figure 5D, a q value cutoff of <0.05 was used.

### Gene ontology (GO) term enrichment analysis

Gene Ontology (GO) term enrichment was performed on differentially expressed genes per individual cluster using the enrichGO function of the clusterProfiler R package (version 3.10.1) (Yu et al., 2012). The cut-off value for significance was set at q<0.05. Redundant terms were removed from the graphical display of the results, and terms displayed in Figure 5E were further filtered for q-value.

### ChIP-Seq data (previously published)

The Hoxd13 ChIP-Seq data was downloaded from GEO (accession number GSE81358), and was described in a previous study (Sheth et al., 2016). Previously published murine FLAGHoxd13 and FLAG-Hoxd13 Q317R ChIP-Seq data in chicken micromass were obtained from GEO (accession number GSE44799).

### ChIP-Seq data processing

The Hoxd13 ChIP-Seq data was aligned to the mm9 genome assembly using bowtie version 1.0.0 (Langmead et al., 2009) with parameters '-n 2 -e 70 -m 1 -k 1 --best -l 200'.

The murine FLAG-Hoxd13 transfected into chicken micromass ChIP-Seq datasets were aligned to the chicken genome (assembly gg3). The paired-end sequencing reads of the murine H3K27Ac ChIP-Seq data with D. *melanogaster* spike-ins were first adapter- and quality trimmed Then reads were aligned to the mouse genome mm9 or fly (*D. melanogaster*) genome.

Genome wide rpm/bp normalized coverage profiles were created by binning the mouse genome into 3mb regions and subsequently using bamToGFF_turbo.py resulting in 50 bp sized bins. Hoxd13 and spdh rpm/bp values for each bin were finally normalized by spike-in factors obtained from the D. *melanogaster* spike-in ChIP-Seq reads (Orlando et al., 2014).

### H3K27Ac ChIP-Seq analysis

In Figure 5J, the fold change in spike-in normalized H3K27Ac ChIP-Seq signal between spdh and wt samples was calculated as follows. First, the nearest Hoxd13 peak within the same TAD around to the TSS of genes that were either downregulated or upregulated in cluster 4 was identified.

### Capture C data

Capture C profiles for hindlimb and midbrain tissues from mouse embryos at the developmental stage E11.5 were obtained from GEO (accession number GSE84795).

### Topologically Associating Domains (TADs)

Topologically associating domains (TADs) in mouse E12.5 limb bud cells were described in a previous study (Kraft et al., 2019).

### Enrichment of Hoxd13 ChIP-Seq peaks around Cluster 4 dysregulated genes

The number of Hoxd13 ChIP-Seq peaks within TADs that contain a Cluster 4 dysregulated gene was calculated as follows. First, TADs that contain the transcription start site (TSS) of at least one gene dysregulated in Cluster 4 (Figure 5G) were identified, and then the number of Hoxd13 peaks within those 82 TADs were calculated (Figure 5G). Refseq annotation catalog 89 (24th Sep. 2018) was used to define TSSs.

### Enrichment of Capture C signal at Hoxd13 peaks

The mean contact frequency between Cluster 4 dysregulated genes and Hoxd13 ChIP-Seq peaks (Figure 5H) was calculated as follows. First, the genes dysregulated in Cluster 4 were identified. 25 of these genes were used as Capture C viewpoints in the Capture C data, and these genes were kept for further analysis. The Hoxd13 peaks within the TADs that contain these 25 genes were identified, and only the Hoxd13 peaks that were separated by at least 50kb from the viewpoint gene were kept for further analysis (to reduce the elevated background signal close to the viewpoints). The summit positions of the Hoxd13 peaks were then identified, and the mean Capture C signal around the summit position were plotted in 5kb bins around the summit (Figure 5H left). As a control, the same genomic co-ordinates of the peaks and bins were used to calculate the mean Capture C signal in embryonic midbrain tissue (Figure 5H right).

### Mean expression value of individual genes

For genes depicted in Figure 5F the coverage signal from the bigWig files of the 11 Clusters was exported. For each gene, the mean coverage of the 3'UTR plus last coding exon was calculated as bins per million mapped reads.

### Repeat expansions in Hoxd13, Hoxa13, Runx2 and TBP

The repeat expansion information displayed in Figure 2B, 6A, 6J, 6S were curated from multiple studies, some of which were summarized in previous reviews (Albrecht and Mundlos, 2005; Darling and Uversky, 2017). The first +7A, +8 and +10 polyA repeat expansions associated with synpolydactyly were described in (Muragaki et al., 1996). Additional expansions, including pedigrees with +9A and +14A mutations were described in (Goodman et al., 1997). Hoxa13 contains three short alanine repeats (14, 12 and 18 alanines, respectively), and hand-footgenital syndrome (HFGS) -associated mutations in all three repeats have been described (Goodman et al., 2000; Innis et al., 2004). TBP polyQ repeat expansions were described in several early reports (Maltecca et al., 2003; Nakamura et al., 2001). The Runx2 alanine expansions were described in (Mundlos et al., 1997).

### IDR classification: data retrieval and preprocessing

Protein sequences for all human RefSeq gene models were downloaded from UCSC (Karolchik et al., 2004). For each protein, the PONDR (disorder) scores (VSL2) were downloaded from PONDR (Peng et al., 2006). An intrinsically disordered region (IDR) was defined as a sequence of at least 50 consecutive amino acids with a PONDR (VSL2) score above 0.5. Two IDRs in the same protein were fused if less than 40 consecutive amino acids with a score below 0.5 separated them. For the classification of TF IDRs (Figure 7A) the longest IDR from all protein isoforms of the same gene was used. The list of transcription factors and their DNA binding domain (DBD) sequences used in Figure 7A were retrieved from a previous study (Lambert et al., 2018). The amino acid composition, GRAVY (hydrophobicity) score, instability score, aliphatic index and isoelectric point (pH(I)) of each DBD and IDR was extracted using the protparam webservice (Artimo et al., 2012). PhyloP conservation scores based on the 100way multiple species alignment with hg19 as the reference species were obtained from UCSC.

### IDR/DBD clustering and annotation

The amino acid composition, aliphatic index, isoelectric point (pl), PONDR, GRAVY and instability scores for each IDR of all TFs was used as input. Principal component analysis (PCA) was performed to identify the most variable features, and the first ten PCs (that explain 80% of the variability of the input data) were subsequently used. The transformed data were then subject to K-means clustering. We determined the Bayesian information criterion (BIC) for various values of "k" and identified k=7 as an optimal cluster number as the infliction point on the BIC plot (Schwarz, 1978). For each TF, the DBD annotation of a previous study was used (Lambert et al., 2018). The presence of a homopolymeric repeat was defined using a minimum repeat length of ten amino acids in the IDR (Figure 7A).

Figure 7A used the R packages circlize (Gu et al., 2014) and dendextend (Galili, 2015) to visualize the identified IDR clusters, DBD clusters and IDR and DBD clusters. The inner circle in Figure 7A are the identified 7 IDR clusters. The middle circle highlights the TF IDRs with a homopolymeric alanine or glutamine expansion in the IDR and the outer circle highlights the transcription factor family a TF IDR belongs to. The FoxP1/2/3 DNA binding motifs in the outsets were obtained from the Jaspar database (Mathelier et al., 2014).

The enrichment analyses of TFs whose IDRs comprise the 7 IDR clusters for categories displayed in Figure 7D was done as follows. The enrichment of Gene Ontology (GO) terms for biological processes was carried out by using the GOrilla webservice (Eden et al., 2009) with two unranked lists. The TFs found in a cluster were used as the target gene set and all TFs were used as the background set. The p-value of 10-3 was kept as a lower bound threshold. In Figure 7D, only GO terms that had an least 15 genes overlap with at least one of the seven IDR clusters, and an FDR of maximum 5% were displayed. Phenotype ontology terms were retrieved from www.human-phenotype-ontology.org (Kohler et al., 2014). Fisher's exact test in R was carried out for each of the TFs in the seven clusters by setting the parameter alternative='greater' and a p-value cutoff of 0.05. The significant phenotype terms were then filtered in the same way as the GO terms. The GWAS associations were obtained from https://www.ebi.ac.uk/gwas/ (Buniello et al., 2019) and we used the exact same processing as for the phenotype ontology terms. The TF annotation of activators and/or repressors (Figure 7D) was obtained by using the R bioconductor package, and the GO.db was queried for terms 'activator' or 'repressor' and intersected with the term 'transcript'. This resulted in 430 activators and 231 repressors.

### 3. RESULTS

### 3.1 The Hoxd13 IDR drives phase separation

Disease-associated repeat expansions are significantly enriched in transcription factors (TFs) (p<10-4, Fisher's test; Figure 1A). To investigate the hypothesis that disease-associated repeat expansions alter the phase separation capacity of the TFs in which they occur, we first focused on the homeobox TF Hoxd13 as a proof of concept, because the genetics of Hoxd13 in disease is well characterized (Albrecht and Mundlos, 2005; Albrecht et al., 2004; Kuss et al., 2009; Muragaki et al., 1996; Villavicencio-Lorini et al., 2010). During mammalian embryogenesis, Hoxd13 is expressed in the limb bud (Figure 1B) and controls skeletal morphogenesis (Villavicencio-Lorini et al., 2010). Hoxd13 mutations cause hereditary limb malformations e.g. synpolydactyly, a syndrome characterized by extra digits and digit fusions (Muragaki et al., 1996). High-resolution confocal imaging of the Hoxd13 protein in mouse limb bud cells revealed that Hoxd13 forms discrete nuclear puncta (Figure 1B). Stochastic optical reconstruction microscopy (STORM) revealed that Hoxd13 puncta in limb bud cells were ^{∼}100nm in size and occurred in less DNA dense parts of the nucleus (Figure 1C), similar to previously described co-activator and RNAPII puncta (Cho et al., 2018). These results are consistent with the notion that Hoxd13 may be a component of transcriptional condensates.

Intrinsically disordered regions (IDRs) in proteins are known to drive phase separation (Banani et al., 2017; Shin and Brangwynne, 2017), and the Hoxd13 N-terminus has sequence features predictive of an IDR (Figure 1D). Therefore, we used an optogenetic platform to investigate whether the Hoxd13 IDR can drive phase separation in vivo. In brief, the optoDroplet assay involves expression of a fusion protein consisting of the IDR of interest, mCherry, and the photolyase domain of the *Arabidopsis thaliana* Cry2 protein. Excitation of Cry2 with blue light stimulates its self-association, which leads to an increase of local concentration of the fused IDR (Shin et al., 2017). IDRs that drive phase separation subsequently facilitate the formation of liquid-like droplets (Figure 1E), which tend not to form in the absence of the IDR (Sabari et al., 2018; Shin et al., 2017). The Hoxd13 IDR fused to mCherry and Cry2 facilitated the formation of droplets upon blue-light stimulation in HEK293T cells (Figure 1F-G). Fluorescence recovery after photobleaching (FRAP) revealed rapid, liquid-like recovery rate of the Hoxd13 IDR-mCherry-Cry2 droplets (Figure 1H), and the droplets were sometimes observed to undergo fusion (Figure 1I), which are characteristics of phase-separated condensates (Alberti et al., 2019). These results suggest that the Hoxd13 IDR has the capacity to drive phase separation in vivo.

If the Hoxd13 IDR can drive phase separation, the purified IDR should form liquid-like droplets in vitro. Purified recombinant Hoxd13 IDR-mCherry fusion protein indeed formed droplets in the presence of 10% PEG-8000, while an mCherry control did not (Figure 1J). As expected for phase-separated condensates (Alberti et al., 2019), the Hoxd13 IDR droplets had spherical shape, and their size scaled with the concentration of the protein (Figure 1J). These results indicate that the Hoxd13 IDR can form condensates in vitro.

### 3.2 Synpolydactyly-associated repeat expansions enhance Hoxd13 IDR phase separation

Expansions of an alanine repeat in the IDR of Hoxd13 cause synpolydactyly, a congenital limb malformation (Figure 2A-B) (Kuss et al., 2009; Muragaki et al., 1996). Of note, Hoxd13 mutants that contain short (e.g. +7A) synpolydactyly-associated expansions have not been described to form aggregates, suggesting that protein aggregation does not explain the pathology of short repeat expansions (Albrecht and Mundlos, 2005; Albrecht et al., 2004; Villavicencio-Lorini et al., 2010). Since the repeat expansions occur within the Hoxd13 IDR, which promotes phase separation, we hypothesized that the repeat expansions may alter the phase separation capacity of the Hoxd13 IDR. Hoxd13 IDRs including the wild type alanine repeat and several synpolydactyly-associated expansions (+7A, +8A, +9A, +14A) were thus tested in the optoDroplet system. To ensure investigation of the phase separation capacity of the Hoxd13 IDR in its nuclear context, an SV40 nuclear localization sequence (NLS) was included in all subsequent fusion constructs. Short expansions enhanced the rate of light-induced droplet formation in live cell nuclei (Figure 2C-D). In addition, spontaneously formed Hoxd13 IDR condensates were observed in cells expressing the +8A, +9A and +14A alleles, and the ratio of the protein in the spontaneous condensates and the soluble (diffuse) fraction correlated with the length of the repeat expansion (Figure 2C). DNA staining confirmed that the spontaneous condensates formed by the +8A, +9A alleles were nuclear, whereas the condensates formed by the +14A allele were cytosolic. These results suggest that alanine repeat expansions enhance the phase-separation capacity of the Hoxd13 IDR.

Phase separated condensates deep beyond the phase boundary can transition to a gel-like state characterized by arrested, yet reversible dynamics (Shin et al., 2017). Consistent with this notion, the light-induced droplets formed by repeat-expanded Hoxd13 IDRs exhibited considerably slower FRAP rates than droplets formed by the wild type IDR (Figure 2E). Transient light stimulation revealed that formation of these droplets was reversible. In addition, the spontaneous condensates formed by the +8A and +9A Hoxd13 IDRs exhibited slow, but detectable FRAP rates (Figure 2F). Co-expression of an mCherry-tagged Hoxd13 +8A IDR, which forms spontaneous condensates, with YFP-tagged Hoxd13 wt and +7A IDRs confirmed that recruitment of IDR fusion proteins to IDR condensates is dependent on the IDR, and correlates with the length of the alanine repeat. These results suggest that the alanine repeat expansion alters the material properties of Hoxd13 IDR optoDroplets.

To further probe the effect of alanine repeat expansion on Hoxd13 phase separation, we purified various recombinant Hoxd13 IDR-mCherry fusion proteins, and investigated their phase separation capacity in droplet formation buffer in vitro. The +7A and +10A IDR mutants formed more, and more concentrated droplets compared to the wt IDR at similar concentrations (Figure 2G-H). Furthermore, the concentrations at which droplets appeared (i.e. the saturation concentration Csat) were lower for +7A and +10A IDRs compared to the wt IDR (Figure 2G-H). Taken together, these results suggest that the alanine repeat expansion enhances phase separation of the Hoxd13 IDR, and are consistent with the previously described correlation between the length of the alanine repeat expansion and disease severity (Kuss et al., 2009; Muragaki et al., 1996).

### 3.3 Synpolydactyly-associated repeat expansions alter the composition of Hoxd13-containing condensates

Recent studies indicate that TFs can form heterotypic condensates with the IDR of the Med1 subunit of the Mediator co-activator in vitro, and that co-condensation of TFs with the Med1 IDR requires the TFs' IDR (Boija et al., 2018). We thus hypothesized that the alanine repeat expansion of the Hoxd13 IDR alters its ability to co-condense with transcriptional co-activators. To test this model, we purified recombinant Med1 IDR-GFP fusion protein and mixed it with various purified Hoxd13 IDR-mCherry fusion proteins. Of note, the condensation behavior of the Med1 IDR is similar to that of purified partial Mediator complex, and the Med1 IDR has thus been a useful surrogate for Mediator condensates in vitro (Boija et al., 2018; Guo et al., 2019; Sabari et al., 2018). We found that heterotypic Med1 IDR-Hoxd13 IDR co-condensates had dramatically altered composition when Hoxd13 IDRs containing synpolydactyly-associated repeat expansions (+7A, +10A) were used in the mixing experiments. The Med1 IDR droplets incorporated the wild type Hoxd13 IDR at several concentrations, and the wild type Hoxd13 IDR exclusively occurred in co-condensates with the Med1 IDR (Figure 3A). On the other hand, +7A and +10A Hoxd13 IDRs were more enriched in Med1 IDR-containing droplets (p<10-15 Welch's t-test), but the heterotypic droplets contained on average significantly less Med1 IDR than wild type Hoxd13 IDR co-condensates (p<10-15 Welch's t-test) (Figure 3A-D). These results suggest that the repeat-expanded Hoxd13 IDR co-condenses with less Med1 IDR than the wild type Hoxd13 IDR, a phenomenon we term "condensate unblending."

We next tested whether various Hoxd13 IDRs unblend from the Med1 IDR even at concentrations at which they form comparable homotypic droplets alone. To this end, we preassembled Med1 IDR-GFP droplets for 30 minutes, and mixed the preassembled Med1 IDR-GFP droplets with various concentrations of Hoxd13 IDR-mCherry fusion proteins. These included concentrations where the Hoxd13 proteins alone formed droplets similar in number, size, and protein content (wt: 5µM, +7A: 1µM, +10A: 0.2µM; see Figure 2H, and Figure 3E insets). The wild type Hoxd13 IDR at 1µM was incorporated in Med1 IDR droplets, and its enrichment was concentration-dependent (Figure 3E-F top two panels). On the other hand, small condensates that consisted of almost exclusively Hoxd13 +7A IDR were apparent in mixtures containing 1µM Hoxd13 +7A IDR, and the median Med1 IDR content of the condensates was substantially lower relative to the Med1 IDR content of the Hoxd13 wt IDRMed1 IDR co-condensates (Figure 3E-F, compare first and third panels). A similar effect was observed when 0.2µM Hoxd13 +10A IDR was used in the mixing experiment (Figure 3E-F). These results suggest that condensate unblending occurs at various concentrations in vitro.

The unblending of repeat-expanded Hoxd13 IDRs from Med1 IDR-containing co-condensates indicates that the alanine repeat expansion increases the preference for homotypic Hoxd13 IDR-IDR interactions over heterotypic interactions with the Med1 IDR. If this model is true, disrupting weak hydrophobic interactions could revert the composition of repeat-expanded Hoxd13 IDR-containing Med1 co-condensates to be more similar to the composition of Hoxd13 wt IDR-Med1 IDR co-condensates (i.e. "reblend" them). To test this notion, we treated Hoxd13 IDR-Med1 IDR co-condensates with ATP, a small hydrotropic molecule known to solubilize hydrophobic molecules at mM concentrations (Patel et al., 2017). ATP reblended Hoxd13 +7A IDR-Med1 IDR co-condensates in a dose dependent manner (Figure 3G-I). The reblending effect appeared specific to ATP, as lipoic acid, lipoamide and mitoxantrone - compounds that were recently reported to dissolve condensates formed by stress granule proteins (Wheeler et al., 2019) -failed to reblend Hoxd13 +7A IDR-Med1 IDR co-condensates (Figure 3J). Taken together these results suggest that Hoxd13 repeat expansion leads to unblending of Hoxd13 IDR-Med1 IDR co-condensates (Figure 3K).

### 3.4 Altered composition and properties of repeat-expanded Hoxd13-condensates in vivo

Next we sought to investigate the effect of disease-associated repeat expansions on endogenous Hoxd13-containing condensates in vivo. To this end, we isolated limb bud cells from homozygous spdh mouse embryos and wild type littermates. Spdh mice carry +7A repeat expanded Hoxd13 alleles, and homozygous spdh mice exhibit synpolydactyly (Bruneau et al., 2001). We first tested whether transcriptional co-activators are associated with Hoxd13-containing condensates in limb bud cells using antibody staining and STORM imaging. As no high quality Mediator antibodies were available to co-stain with Hoxd13 in STORM, Brd4 was visualized instead of Mediator. Brd4 is a ubiquitous co-activator that co-purifies with Mediator (Jiang et al., 1998), co-condenses with Mediator (Sabari et al., 2018), and Brd4 chemical inhibition dissolves Mediator condensates in vivo (Cho et al., 2018). We found that Brd4 more frequently overlapped Hoxd13 puncta in wild type limb bud cells than in spdh limb bud cells harboring Hoxd13 +7A alleles [1.5-fold difference in Manders co-efficient, p-value<0.001, twotailed t-test)] (Figure 4A-B). As a control, HP1alpha was co-visualized with Hoxd13, and the two proteins showed negligible overlap (Figure 4B). These results suggest that Hoxd13-condensates have altered composition in vivo.

To investigate the biophysical properties of Hoxd13-containing condensates in vivo, wt and spdh limb bud cells were treated with 1,6-hexanediol (1,6-HD), a short-chain aliphatic alcohol that dissolves various intracellular membraneless organelles (Boehning et al., 2018; Chong et al., 2018; Sabari et al., 2018). Hoxd13 was subsequently visualized with immunofluorescence. Both wt and +7A Hoxd13 localized within discrete nuclear puncta (Figure 4D), but the puncta detected in limb bud cells expressing +7A Hoxd13 displayed considerably reduced sensitivity to 1,6-HD (Figure 4D-E). These results suggest that a short alanine repeat expansion (+7A) alters the biophysical properties of Hoxd13-containing puncta in limb bud cells.

To test whether repeat-expansion impairs the ability of Hoxd13 condensates to recruit Mediator in vivo, various Hoxd13 IDRs were tethered to a LacO array in U2OS cells expressing an ectopic Med1 IDR-YFP fusion protein (Figure 4E) (Janicki et al., 2004). Med1 IDR-YFP was found mildly enriched at the LacO array occupied by the Hoxd13 wt IDR tether, and its incorporation was significantly reduced in the +7A and +10A Hoxd13 IDR tethers (P<10-2, Welch's t test) (Figure 4E-F). Consistent with the notion that Hoxd13 repeat expansion impairs Mediator recruitment to Hoxd13 condensates, spontaneously forming Hoxd13 +8A IDR condensates observed in HEK293T cells expressing the Opto-Hoxd13 IDR constructs excluded the Med1 IDR.

The Mediator co-activator plays key roles in recruiting RNA Polymerase II to TF-bound genes (Levine et al., 2014), so a reduction of Med1 content in mutant Hoxd13-containing condensates would be expected to reduce the transcriptional activity of repeat-expanded Hoxd13. Consistent with this idea, repeat-expanded Hoxd13 alleles displayed reduced activity in luciferase reporter assays (Figure 4G).

To rule out that the repeat expansion affects DNA binding of Hoxd13, we performed Chromatin Immunoprecipitation followed by sequencing (ChIP-Seq) on FLAG-tagged Hoxd13 proteins expressed in a chicken transgenic cell system (Ibrahim et al., 2013).

Taken together, these results suggest that repeat expansions alter the composition and biophysical properties of Hoxd13-containing condensates in disease-relevant limb bud cells and transgenic cell systems, and reduce Hoxd13-dependent transcriptional activity without affecting DNA binding.

### 3.5 Hoxd13 repeat expansion alters the transcriptional program of several cell types in a cell-specific manner

The condensate unblending model predicts that the Hoxd13 repeat expansion alters the composition and properties of Hoxd13-containing transcriptional condensates, which leads to deregulated gene expression programs. To comprehensively assess the impact of Hoxd13 repeat expansion on gene expression in disease-relevant cells, we performed single cell RNASeq (scRNA-Seq) on limb buds from mouse embryos that carry either wild type or +7A repeat-expanded Hoxd13 alleles (spdh mice). In total, 9,655 single cell transcriptomes were captured, with on average >60,000 transcripts and ^{∼}3,500 genes detected per cell. The single cell transcriptomes of 4,464 wild type cells were clustered to generate a reference map of cell states in the limb bud (Figure 5A-C) and 4,147 spdh limb bud cells were assigned to the 11 wild type cell states (Figure 5A-C). These analyses revealed that +7A Hoxd13 affected the abundance primarily of two cell types: interdigital mesenchymal cells were depleted, and proximal chondrocytes were enriched in the spdh limb bud (Figure 5C), consistent with previous observations (Kuss et al., 2009; Villavicencio-Lorini et al., 2010).

Examination of dysregulated genes within individual cell states revealed cell type-specific changes in the transcriptional program of several cell types in the spdh limb. For example, genes associated with mesenchyme differentiation (e.g. *Msx1, Msx2, Tgfb2*) and digit morphogenesis (e.g. *Hoxd12, Hoxd13*) were downregulated in the spdh interdigital mesenchymal cells (Cluster 4; whose abundance was lower in the spdh limbs) (Figure 5D-E). On the other hand, the transcriptome profile of proximal chondrocytes (Cluster 6), did not substantially change (Figure 5D). Hoxd13 ChIP-Seq in wt limbs confirmed that the topologically associating domains (TADs) containing genes dysregulated in the interdigital mesenchyme cells were significantly enriched for Hoxd13 binding (p<10-5 Wilcoxon test) (Figure 5F-H). Capture C (chromosome conformation) data in wild type limb bud cells confirmed interactions between the Hoxd13-bound elements and those genes (Figure 5F, 5H). We also performed ChIP-RX for the transcription-associated H3K27Ac modification in wt and spdh limb buds, and found a significant reduction of H3K27Ac at the nearest Hoxd13-bound sites around the genes downregulated in interdigital mesenchymal cells (p=0.03, Wilcoxon test) (Figure 5I-J). These results suggest that Hoxd13 repeat expansion leads to reduced transcription of key differentiation Hoxd13-target genes in interdigital mesenchymal cells associated with the synpolydactyly phenotype.

### 3.6 Disease-associated repeat expansions alter the phase separation capacity of other TFs

Amino acid repeat expansions in TFs occur in various diseases (Figure 1A). We therefore investigated whether the phase separation capacity of other TFs may be altered by disease-associated repeat expansions.

Hoxa13 is a homeobox TF involved in vertebrate limb-and urogenital tract development (Albrecht and Mundlos, 2005), and alanine repeat expansions in the Hoxa13 IDR cause hand-foot-genital syndrome (HFGS) (Figure 6A) (Goodman et al., 2000). The Hoxa13 IDR facilitated phase separation in the optoDroplet system (Figure 6B-C), and Hoxa13 IDR droplets exhibited liquid-like FRAP rate (Figure 6D). The Hoxa13 IDR containing a short (+7A) HFGSlinked expansion tended to form aggregates with negligible FRAP rate (Figure 6B, 6D). Furthermore, the +7A expansion enhanced droplet formation of mCherry-tagged, purified Hoxa13 IDR in vitro (Figure 6E-F), and lead to unblending of Hoxa13 IDR from Med1-IDR co-condensates (Figure 6G-H). Last, the +7A expansion significantly reduced transcriptional activity of the Hoxa13 IDR fused to a Gal4 DNA-binding domain (DBD) in a luciferase reporter system (p<10-3, two tailed t-test) (Figure 6I). These results suggest that the Hoxa13 IDR can drive phase separation, and that a pathological alanine repeat expansion alters its phase separation capacity, co-condensation with the Med1 IDR, and transcriptional activity.

Runx2 is a RUNT family TF that controls bone morphogenesis, and expansions of a short alanine and glutamine repeat in the Runx2 IDR are associated with cleidocranial dysplasia (CCD), a disorder of severe skeletal defects (Figure 6J) (Mastushita et al., 2015; Shibata et al., 2016). The Runx2 IDR facilitated phase separation in the optoDroplet system (Figure 6K-L,), and Runx2 IDR droplets exhibited liquid-like FRAP rate (Figure 6M). The Runx2 IDR containing a CCD-associated alanine expansion a (+10A) tended to form solid aggregates with negligible FRAP rate (Figure 6K, 6M). Furthermore, the +10A expansion enhanced droplet formation of mCherry-tagged, purified Runx2 IDR in vitro (Figure 6N-O), and lead to unblending of Runx2 IDR from Med1-IDR co-condensates (Figure 6P-Q). Last, the +10A expansion significantly reduced transcriptional activity of the Runx2 IDR fused to a Gal4 DNA-binding domain (DBD) in a luciferase reporter system (p<10-3, two tailed t-test) (Figure 6R). These results suggest that the Runx2 IDR can drive phase separation, and that a pathological alanine repeat expansion alters its phase separation capacity, co-condensation with the Med1 IDR, and transcriptional activity.

The TATA-box binding factor TBP is a highly conserved general transcription factor that plays a key role in transcription initiation. The TBP N-terminus contains a polymorphic polyglutamine (Q) repeat, whose typical size ranges between 24-42 glutamines, and repeats consisting of >46 glutamines are linked to spinocerebellar ataxia type 17 (SCA17), a progressive neurodegenerative disease (Nakamura et al., 2001). A TBP IDR with wild type glutamine repeat length (38Q) facilitated phase separation over the Cry2 control in the optoDroplet system (Figure 6T-U), and TBP IDR droplets exhibited liquid-like FRAP rate (Figure 6V). In contrast, an SCA17-associated polyglutamine repeat expansion (53Q) inhibited TBP IDR droplet formation (Figure 6T-U). These results suggest that the TBP IDR can drive phase separation, and its phase separation capacity is inhibited by pathological glutamine repeat expansion.

### 3.7 A catalog of human transcription factor IDRs

Transcription factors (TFs) typically consist of highly structured DNA binding domains (DBD) and intrinsically disordered activation domains (ADs/IDRs) (Lambert et al., 2018; Mitchell and 10 Tjian, 1989; Staby et al., 2017). TF IDRs are low complexity protein sequences, several of which are known to drive phase separation (Boija et al., 2018; Chong et al., 2018). TF IDRs frequently contain homopolymeric repeats (proline, serine, alanine, glycine, glutamine, histidine), but only expansions of alanine and glutamine repeats have been linked to human pathologies to date. We thus reasoned that various molecular features of TF IDRs dictate their phase separation capacity. To gain insights into those features, we created a catalog of IDRs in ^{∼}1,500 human TFs. We first identified IDRs and DBDs in human TFs, and clustered them based on amino acid composition, hydrophobicity, aliphatic index, stability, isoelectric point and disorder. The clustering algorithm separated DBDs and IDRs with 96% efficiency, and could even separate DBD families previously annotated based on structural homology (Lambert et al., 2018). The TF IDRs were broadly distributed in seven major clusters based on various features (Figure 7A), and were on average more disordered and less conserved than DBDs (Figure 7B-C). The IDRs in individual clusters belong to TFs from diverse DBD families, and TFs that have similar DBDs contain IDRs that belong to diverse IDR clusters. For example, 3/4 FoxP family members have a cluster 6 IDR, whereas FoxP3 has a cluster 1 IDR, and the DNA-binding specificity of these TFs is virtually identical (Figure 7A). A notable but expected exception was IDR cluster 5, which predominantly consisted of the KRAB-Zinc Finger TFs (Figure 7A). Overall, TFs in diverse IDR clusters were enriched for the presence of various homopolymeric repeats, and were associated with a spectrum of biological processes, human phenotypes and disease-associated genetic variants (Figure 7A, 7D).

One key feature of cluster 1 IDRs was high alanine content, and this cluster included 32 of the 33 poly-alanine-containing IDRs (p<10-16, Fisher's exact test) (Figure 7A). We thus hypothesized that the hydrophobic alanine residues may drive the phase separation capacity of cluster 1 IDRs. To test this model, we engineered various repeat-deletion mutant (-7A, -15A) Hoxd13 IDRs, an IDR in which the negatively charged residues were deleted (DEdel), and tested their phase separation capacity in the optoDroplet system (Figure 7E). Deletion of 7 and 15 alanines, which decreased hydrophobicity, inhibited droplet formation, while deletion of the negatively charged residues enhanced droplet formation by the Hoxd13 IDR (Figure 7EG). We then selected 10 Cluster 1 IDRs, and tested the contribution of the poly-alanines to transcriptional activity in the Gal4 DBD-luciferase system. 5/10 IDRs functioned as activators in this minimal system, and for 4/5 of the activators, deletion of the alanine repeat enhanced reporter activity (Figure 7H). Analysis of further alanine mutants revealed that the reporter activity of Hoxa13 IDR negatively correlated with the number of alanines (Figure 7I). These results suggest that the alanine repeat drives homotypic phase separation of Cluster 1 IDRs, and that poly-alanine -driven TF phase separation can inhibit transactivation.

### 4. DISCUSSION

We found that repeat expansions in intrinsically disordered regions (IDRs) of transcription factors (TFs) alter the phase separation capacity of those TFs, and their capacity to form transcriptional condensates with components of the transcription machinery. The IDR of the HOXD13 TF drives phase separation and co-condensation with the Mediator co-activator. Synpolydactyly-associated alanine repeat expansions facilitate homotypic Hoxd13 phase separation, and the mutant Hoxd13 IDRs co-condense less readily with Mediator than the wild type HOXD13 IDR, a phenomenon we term "condensate unblending". In a mouse synpolydactyly model, HOXD13 repeat expansion led to cell-type specific transcriptional changes of HOXD13 targets in disease-relevant cells. Thus, disease-associated mutations in TF IDRs alter the features that drive the TF's condensation behavior, and present a molecular classification of TF IDRs as a framework to dissect the pathomechanism of diseases associated with transcription factor dysfunction. The results presented here support a model that disease-associated amino acid repeat expansions in TF IDRs alter the TFs' phase separation capacity, and their ability to co-condense with transcriptional coactivators. For the Hoxd13 TF, synpolydactyly-associated alanine repeat expansions were found to enhance the phase separation capacity of the Hoxd13 IDR, and the mutant Hoxd13 IDR was able to co-condense with dramatically less Mediator than the wild type IDR, a phenomenon we term "condensate unblending" (Figure 3K). In a mouse model of synpolydactyly, a Hoxd13 repeat expansion altered the co-activator content and biophysical properties of Hoxd13-containing condensates, and led to cell type-specific transcriptional changes of Hoxd13 target genes in disease-relevant cells. For Hoxa13 and Runx2, disease11 associated alanine repeat expansions similarly enhanced homotypic phase separation and led to unblending from Mediator, while for TBP, a disease-associated glutamine repeat expansion reduced the phase separation capacity of its IDR. We propose that aberrant phase separation of TFs and unblending of transcriptional condensates may underlie human pathologies associated with mutations in the IDRs of transcriptional regulators.

Several lines of evidence indicate that altered phase separation underpins the effect of Hoxd13 repeat expansions. The saturation concentration Csat (at which condensates are observed) is lower for purified mutant Hoxd13 IDRs than the wt IDR (Figure 2G-H), and the mutations enhance condensate formation in the optoDroplet system (Figure 2C-D). The lower Csat of mutant Hoxd13 IDRs is in turn associated with an increase in TF IDR content and reduced Med1 IDR content of heterotypic co-condensates in vitro (Figure 3A-F), and reduced coactivator- Hoxd13 association in vivo (Figure 4A-B). This effect is consistent with recent reports that heterotypic interactions dominate phase separation of endogenous condensates, and that Csat of heterotypic condensates can be modulated by physico-chemical properties of their components (Choi et al., 2019; Riback et al., 2019). Furthermore, the condensate unblending model may help explain why the (+7A) repeat expanded Hoxd13 allele is genetically a dominant negative allele (Albrecht and Mundlos, 2005; Villavicencio-Lorini et al., 2010), why the phenotype of repeat expansions is distinct from the phenotype of Hoxd13 deactivating mutations (Bruneau et al., 2001; Dolle et al., 1993), and why the length of the repeat expansion correlates with disease severity (Goodman et al., 1997).

Repeat expansion diseases include severe, incurable neurodegenerative and developmental diseases typically associated with the presence of large protein aggregates (Albrecht and Mundlos, 2005; Orr and Zoghbi, 2007; Zoghbi and Orr, 2000). The condensate model presented here may explain several observations of the pathology of repeat expansion diseases that are less readily explained by toxicity of aggregates. For example, for poly-alanine expansions that occur in TFs, aggregates or nuclear inclusions were only described in overexpression systems but not in primary tissue to date (Albrecht and Mundlos, 2005; Albrecht et al., 2004; Villavicencio-Lorini et al., 2010). Furthermore, the short +7A repeat expansion of Hoxd13 ecapitulates the human synpolydactyly phenotype in mice, yet without any evidence of protein aggregation in the limb bud (Albrecht et al., 2004; Villavicencio-Lorini et al., 2010). For polyglutamine expansion diseases, e.g. Huntington's disease, previous studies have established a correlation between aggregate formation by the mutant huntingtin (htt) protein and degeneration of specific neurons (Davies et al., 1997; Ross and Poirier, 2004). However, manipulation of the Ubiquitin-proteasome pathway was reported to decrease aggregate formation without decreasing toxicity of the repeat-expanded htt protein in primary cell models, suggesting that the soluble fraction of the protein may be responsible for its cytotoxic effects (Saudou et al., 1998; Truant et al., 2008). Changes in the phase separation capacity, and miscibility of the repeat expanded proteins in heterotypic condensates are consistent with these diverse observations.

Disease-associated repeat expansions occur not just in transcription factors, but in other nuclear proteins e.g. the ataxin genes, several of which have been linked to chromatin regulation and transcription (Albrecht and Mundlos, 2005; Darling and Uversky, 2017; La Spada and Taylor, 2010; Orr and Zoghbi, 2007). It is thus plausible that disease-associated changes may alter the phase separation capacity and miscibility of various nuclear proteins within heterotypic condensates, which in turn perturbs cellular gene expression programs. Mutations in the IDR of cellular proteins, and repeat expansions in RNA have indeed been recently linked to altered phase separation (Jain and Vale, 2017; Meyer et al., 2018; Molliex et al., 2015; Patel et al., 2015). Dysregulated phase separation may thus underlie a wide spectrum of diseases.

The results presented here also provide insights into the function of TF activation domains (ADs). TF ADs are typically intrinsically disordered sequences that interact with components of the transcription machinery, and recent studies suggest that various TF ADs (i.e. IDRs) have the capacity to phase separate and to co-condense with the Mediator co-activator in vitro, though the functional importance of these observation has been unclear (Boija et al., 2018; Chong et al., 2018). The results presented here provide evidence for the importance of biochemical characteristics that drive TF phase separation for normal TF function in vivo. Eukaryotic genomes tend to encode hundreds of TFs, whose ADs have limited sequence-level similarity, but many ADs are nevertheless functionally interchangeable (Lambert et al., 2018; Mitchell and Tjian, 1989; Staby et al., 2017; Stampfel et al., 2015). A key outstanding question is whether the interchangeability of TF IDRs is determined by their phase separation capacity, and whether the phase separation capacity of interchangeable TF IDRs has a shared molecular basis. The phase separation of the IDR of the FUS RNA-binding protein, for example, is predominantly driven by cation-pi interactions between tyrosine and arginine residues (Wang et al., 2018), while the phase separation capacity of the Oct4 and Gcn4 TFs' IDRs is predominantly driven by negatively charged residues (Boija et al., 2018). Our draft catalog of human TF IDRs indicates that at least for a subset of TFs, e.g. Hoxd13, the phase separation capacity may be determined by alanine residues, and that different TF IDR clusters correlate with various biological functions and phenotypic effects. Future work into the molecular basis of TF phase separation, and how developmental signaling, post-translational modifications and genetic variants impact the miscibility of TFs in heterotypic condensates with components of the transcriptional machinery may thus open new condensate-directed therapeutic avenues for diseases associated with transcriptional dysregulation.

### 5. REFERENCES

Alberti, S., Gladfelter, A., and Mittag, T. (2019). Considerations and Challenges in Studying Liquid-Liquid Phase Separation and Biomolecular Condensates. Cell 176, 419-434.
Albrecht, A., and Mundlos, S. (2005). The other trinucleotide repeat: polyalanine expansion disorders. Curr Opin Genet Dev 15, 285-293.
Albrecht, A.N., Kornak, U., Boddrich, A., Suring, K., Robinson, P.N., Stiege, A.C., Lurz, R., Stricker, S., Wanker, E.E., and Mundlos, S. (2004). A molecular pathogenesis for transcription factor associated polyalanine tract expansions. Human molecular genetics 13, 2351-2359.
Artimo, P., Jonnalagedda, M., Arnold, K., Baratin, D., Csardi, G., de Castro, E., Duvaud, S., Flegel, V., Fortier, A., Gasteiger, E., et al. (2012). ExPASy: SIB bioinformatics resource portal. Nucleic acids research 40, W597-603.
Banani, S.F., Lee, H.O., Hyman, A.A., and Rosen, M.K. (2017). Biomolecular condensates: organizers of cellular biochemistry. Nature reviews Molecular cell biology.
Bi, W., Deng, J.M., Zhang, Z., Behringer, R.R., and de Crombrugghe, B. (1999). Sox9 is required for cartilage formation. Nature genetics 22, 85-89.
Blondel, V.D., Guillaume, J.L., Lambiotte, R., and Lefebvre, E. (2008). Fast unfolding of communities in large networks. Journal of Statistical Mechanics.
Boehning, M., Dugast-Darzacq, C., Rankovic, M., Hansen, A.S., Yu, T., Marie-Nelly, H., McSwiggen, D.T., Kokic, G., Dailey, G.M., Cramer, P., et al. (2018). RNA polymerase II clustering through carboxyterminal domain phase separation. Nature structural & molecular biology 25, 833-840.
Boija, A., Klein, I.A., Sabari, B.R., Dall'Agnese, A., Coffey, E.L., Zamudio, A.V., Li, C.H., Shrinivas, K., Manteiga, J.C., Hannett, N.M., et al. (2018). Transcription Factors Activate Genes through the Phase-Separation Capacity of Their Activation Domains. Cell 175, 1842-1855 e1816.
Bruneau, S., Johnson, K.R., Yamamoto, M., Kuroiwa, A., and Duboule, D. (2001). The mouse Hoxd13(spdh) mutation, a polyalanine expansion similar to human type II synpolydactyly (SPD), disrupts the function but not the expression of other Hoxd genes. Developmental biology 237, 345-353.
Buniello, A., MacArthur, J.A.L., Cerezo, M., Harris, L.W., Hayhurst, J., Malangone, C., McMahon, A., Morales, J., Mountjoy, E., Sollis, E., et al. (2019). The NHGRI-EBI GWAS Catalog of published genomewide association studies, targeted arrays and summary statistics 2019. Nucleic acids research 47, D1005-D1012.
Butler, A., Hoffman, P., Smibert, P., Papalexi, E., and Satija, R. (2018). Integrating single-cell transcriptomic data across different conditions, technologies, and species. Nature biotechnology 36, 411-420.
Cho, W.K., Spille, J.H., Hecht, M., Lee, C., Li, C., Grube, V., and Cisse, II (2018). Mediator and RNA polymerase II clusters associate in transcription-dependent condensates. Science.
Choi, J.M., Dar, F., and Pappu, R.V. (2019). LASSI: A lattice model for simulating phase transitions of multivalent proteins. PLoS computational biology 15, e1007028.
Chong, S., Dugast-Darzacq, C., Liu, Z., Dong, P., Dailey, G.M., Cattoglio, C., Heckert, A., Banala, S., Lavis, L., Darzacq, X., et al. (2018). Imaging dynamic and selective low-complexity domain interactions that control gene transcription. Science 361.
Darling, A.L., and Uversky, V.N. (2017). Intrinsic Disorder in Proteins with Pathogenic Repeat Expansions. Molecules 22.
Davies, S.W., Turmaine, M., Cozens, B.A., DiFiglia, M., Sharp, A.H., Ross, C.A., Scherzinger, E., Wanker, E.E., Mangiarini, L., and Bates, G.P. (1997). Formation of neuronal intranuclear inclusions underlies the neurological dysfunction in mice transgenic for the HD mutation. Cell 90, 537-548.
Dolle, P., Dierich, A., LeMeur, M., Schimmang, T., Schuhbaur, B., Chambon, P., and Duboule, D. (1993). Disruption of the Hoxd-13 gene induces localized heterochrony leading to mice with neotenic limbs. Cell 75, 431-441.
Eden, E., Navon, R., Steinfeld, I., Lipson, D., and Yakhini, Z. (2009). GOrilla: a tool for discovery and visualization of enriched GO terms in ranked gene lists. BMC Bioinformatics 10, 48.
Endesfelder, U., Malkusch, S., Fricke, F., and Heilemann, M. (2014). A simple method to estimate the average localization precision of a single-molecule localization microscopy experiment. Histochem Cell Biol 141, 629-638.
Fabricius, V., Lefebre, J., Geertsema, H., Marino, S.F., and Ewers, H. (2018). Rapid and efficient C terminal labeling of nanobodies for DNA-PAINT. Journal of Physics D: Applied Physics.
Galili, T. (2015). dendextend: an R package for visualizing, adjusting and comparing trees of hierarchical clustering. Bioinformatics 31, 3718-3720.
Goodman, F.R., Bacchelli, C., Brady, A.F., Brueton, L.A., Fryns, J.P., Mortlock, D.P., Innis, J.W., Holmes, L.B., Donnenfeld, A.E., Feingold, M., et al. (2000). Novel HOXA13 mutations and the phenotypic spectrum of hand-foot-genital syndrome. American journal of human genetics 67, 197-202.
Goodman, F.R., Mundlos, S., Muragaki, Y., Donnai, D., Giovannucci-Uzielli, M.L., Lapi, E., Majewski, F., McGaughran, J., McKeown, C., Reardon, W., et al. (1997). Synpolydactyly phenotypes correlate with size of expansions in HOXD13 polyalanine tract. Proceedings of the National Academy of Sciences of the United States of America 94, 7458-7463.
Gu, Z., Gu, L., Eils, R., Schlesner, M., and Brors, B. (2014). circlize Implements and enhances circular visualization in R. Bioinformatics 30, 2811-2812.
Guo, Y.E., Manteiga, J.C., Henninger, J.E., Sabari, B.R., Dall'Agnese, A., Hannett, N.M., Spille, J.H., Afeyan, L.K., Zamudio, A.V., Shrinivas, K., et al. (2019). Pol II phosphorylation regulates a switch between transcriptional and splicing condensates. Nature 572, 543-548.
Hnisz, D., Shrinivas, K., Young, R.A., Chakraborty, A.K., and Sharp, P.A. (2017). A Phase Separation Model for Transcriptional Control. Cell 169, 13-23.
Ibrahim, D.M., Hansen, P., Rodelsperger, C., Stiege, A.C., Doelken, S.C., Horn, D., Jager, M., Janetzki, C., Krawitz, P., Leschik, G., et al. (2013). Distinct global shifts in genomic binding profiles of limb malformation-associated HOXD13 mutations. Genome research 23, 2091-2102.
Innis, J.W., Mortlock, D., Chen, Z., Ludwig, M., Williams, M.E., Williams, T.M., Doyle, C.D., Shao, Z., Glynn, M., Mikulic, D., et al. (2004). Polyalanine expansion in HOXA13: three new affected families and the molecular consequences in a mouse model. Human molecular genetics 13, 2841-2851.
Jain, A., and Vale, R.D. (2017). RNA phase transitions in repeat expansion disorders. Nature 546, 243-247.
Janicki, S.M., Tsukamoto, T., Salghetti, S.E., Tansey, W.P., Sachidanandam, R., Prasanth, K.V., Ried, T., Shav-Tal, Y., Bertrand, E., Singer, R.H., et al. (2004). From silencing to gene expression: real-time analysis in single cells. Cell 116, 683-698.
Jiang, Y.W., Veschambre, P., Erdjument-Bromage, H., Tempst, P., Conaway, J.W., Conaway, R.C., and Kornberg, R.D. (1998). Mammalian mediator of transcriptional regulation and its possible role as an endpoint of signal transduction pathways. Proceedings of the National Academy of Sciences of the United States of America 95, 8538-8543.
Karolchik, D., Hinrichs, A.S., Furey, T.S., Roskin, K.M., Sugnet, C.W., Haussler, D., and Kent, W.J. (2004). The UCSC Table Browser data retrieval tool. Nucleic acids research 32, D493-496.
Kohler, S., Doelken, S.C., Mungall, C.J., Bauer, S., Firth, H.V., Bailleul-Forestier, I., Black, G.C., Brown, D.L., Brudno, M., Campbell, J., et al. (2014). The Human Phenotype Ontology project: linking molecular biology and disease through phenotype data. Nucleic acids research 42, D966-974.
Kraft, K., Magg, A., Heinrich, V., Riemenschneider, C., Schopflin, R., Markowski, J., Ibrahim, D.M., Acuna-Hidalgo, R., Despang, A., Andrey, G., et al. (2019). Serial genomic inversions induce tissue specific architectural stripes, gene misexpression and congenital malformations. Nature cell biology 21, 305-310.
Kuss, P., Villavicencio-Lorini, P., Witte, F., Klose, J., Albrecht, A.N., Seemann, P., Hecht, J., and Mundlos, S. (2009). Mutant Hoxd13 induces extra digits in a mouse model of synpolydactyly directly and by decreasing retinoic acid synthesis. The Journal of clinical investigation 119, 146-156.
Kwon, I., Kato, M., Xiang, S., Wu, L., Theodoropoulos, P., Mirzaei, H., Han, T., Xie, S., Corden, J.L., and McKnight, S.L. (2013). Phosphorylation-regulated binding of RNA polymerase II to fibrous polymers of low-complexity domains. Cell 155, 1049-1060.
La Spada, A.R., and Taylor, J.P. (2010). Repeat expansion disease: progress and puzzles in disease pathogenesis. Nature reviews Genetics 11, 247-258.
Lambert, S.A., Jolma, A., Campitelli, L.F., Das, P.K., Yin, Y., Albu, M., Chen, X., Taipale, J., Hughes, T.R., and Weirauch, M.T. (2018). The Human Transcription Factors. Cell 175, 598-599.
Langmead, B., Trapnell, C., Pop, M., and Salzberg, S.L. (2009). Ultrafast and memory-efficient alignment of short DNA sequences to the human genome. Genome biology 10, R25.
Levine, M., Cattoglio, C., and Tjian, R. (2014). Looping back to leap forward: transcription enters a new era. Cell 157, 13-25.
Li, J., Dong, A., Saydaminova, K., Chang, H., Wang, G., Ochiai, H., Yamamoto, T., and Pertsinidis, A. (2019). Single-Molecule Nanoscopy Elucidates RNA Polymerase II Transcription at Single Genes in Live Cells. Cell 178, 491-506 e428.
Lu, H., Yu, D., Hansen, A.S., Ganguly, S., Liu, R., Heckert, A., Darzacq, X., and Zhou, Q. (2018). Phaseseparation mechanism for C-terminal hyperphosphorylation of RNA polymerase II. Nature 558, 318-323.
Maltecca, F., Filla, A., Castaldo, I., Coppola, G., Fragassi, N.A., Carella, M., Bruni, A., Cocozza, S., Casari, G., Servadio, A., et al. (2003). Intergenerational instability and marked anticipation in SCA-17. Neurology 61, 1441-1443.
Mastushita, M., Kitoh, H., Subasioglu, A., Kurt Colak, F., Dundar, M., Mishima, K., Nishida, Y., and Ishiguro, N. (2015). A Glutamine Repeat Variant of the RUNX2 Gene Causes Cleidocranial Dysplasia. Mol Syndromol 6, 50-53.
Mathelier, A., Zhao, X., Zhang, A.W., Parcy, F., Worsley-Hunt, R., Arenillas, D.J., Buchman, S., Chen, C.Y., Chou, A., lenasescu, H., et al. (2014). JASPAR 2014: an extensively expanded and updated open access database of transcription factor binding profiles. Nucleic acids research 42, D142-147.
Meyer, K., Kirchner, M., Uyar, B., Cheng, J.Y., Russo, G., Hernandez-Miranda, L.R., Szymborska, A., Zauber, H., Rudolph, I.M., Willnow, T.E., et al. (2018). Mutations in Disordered Regions Can Cause Disease by Creating Dileucine Motifs. Cell 175, 239-253 e217.
Mitchell, P.J., and Tjian, R. (1989). Transcriptional regulation in mammalian cells by sequence-specific DNA binding proteins. Science 245, 371-378.
Molliex, A., Temirov, J., Lee, J., Coughlin, M., Kanagaraj, A.P., Kim, H.J., Mittag, T., and Taylor, J.P. (2015). Phase separation by low complexity domains promotes stress granule assembly and drives pathological fibrillization. Cell 163, 123-133.
Mundlos, S., Otto, F., Mundlos, C., Mulliken, J.B., Aylsworth, A.S., Albright, S., Lindhout, D., Cole, W.G., Henn, W., Knoll, J.H., et al. (1997). Mutations involving the transcription factor CBFA1 cause cleidocranial dysplasia. Cell 89, 773-779.
Muragaki, Y., Mundlos, S., Upton, J., and Olsen, B.R. (1996). Altered growth and branching patterns in synpolydactyly caused by mutations in HOXD13. Science 272, 548-551.
Nakamura, K., Jeong, S.Y., Uchihara, T., Anno, M., Nagashima, K., Nagashima, T., Ikeda, S., Tsuji, S., and Kanazawa, I. (2001). SCA17, a novel autosomal dominant cerebellar ataxia caused by an expanded polyglutamine in TATA-binding protein. Human molecular genetics 10, 1441-1448.
Nihongaki, Y., Yamamoto, S., Kawano, F., Suzuki, H., and Sato, M. (2015). CRISPR-Cas9-based photoactivatable transcription system. Chem Biol 22, 169-174.
Orlando, D.A., Chen, M.W., Brown, V.E., Solanki, S., Choi, Y.J., Olson, E.R., Fritz, C.C., Bradner, J.E., and Guenther, M.G. (2014). Quantitative ChIP-Seq normalization reveals global modulation of the epigenome. Cell reports 9, 1163-1170.
Orr, H.T., and Zoghbi, H.Y. (2007). Trinucleotide repeat disorders. Annu Rev Neurosci 30, 575-621.
Patel, A., Lee, H.O., Jawerth, L., Maharana, S., Jahnel, M., Hein, M.Y., Stoynov, S., Mahamid, J., Saha, S., Franzmann, T.M., et al. (2015). A Liquid-to-Solid Phase Transition of the ALS Protein FUS Accelerated by Disease Mutation. Cell 162, 1066-1077.
Patel, A., Malinovska, L., Saha, S., Wang, J., Alberti, S., Krishnan, Y., and Hyman, A.A. (2017). ATP as a biological hydrotrope. Science 356, 753-756.
Peng, K., Radivojac, P., Vucetic, S., Dunker, A.K., and Obradovic, Z. (2006). Length-dependent prediction of protein intrinsic disorder. BMC Bioinformatics 7, 208.
Riback, J.A., Zhu, L., Ferrolino, M.C., Tolbert, M., Mitrea, D.M., Sanders, D.W., Wei, M.T., Kriwacki, R.W., and Brangwynne, C.P. (2019). Composition dependent phase separation underlies directional flux through the nucleolus. BioRxiv, http://dx.doi.org/10.1101/809210.
Ross, C.A. (2002). Polyglutamine pathogenesis: emergence of unifying mechanisms for Huntington's disease and related disorders. Neuron 35, 819-822.
Ross, C.A., and Poirier, M.A. (2004). Protein aggregation and neurodegenerative disease. Nat Med 10 Suppl, S10-17.
Sabari, B.R., Dall'Agnese, A., Boija, A., Klein, I.A., Coffey, E.L., Shrinivas, K., Abraham, B.J., Hannett, N.M., Zamudio, A.V., Manteiga, J.C., et al. (2018). Coactivator condensation at super-enhancers links phase separation and gene control. Science.
Saudou, F., Finkbeiner, S., Devys, D., and Greenberg, M.E. (1998). Huntingtin acts in the nucleus to induce apoptosis but death does not correlate with the formation of intranuclear inclusions. Cell 95, 55-66.
Schnitzbauer, J., Strauss, M.T., Schlichthaerle, T., Schueder, F., and Jungmann, R. (2017). Superresolution microscopy with DNA-PAINT. Nature protocols 12, 1198-1228.
Schwarz, G.E. (1978). Estimating the dimension of a model. Annals of Statistics.
Sheth, R., Barozzi, I., Langlais, D., Osterwalder, M., Nemec, S., Carlson, H.L., Stadler, H.S., Visel, A., Drouin, J., and Kmita, M. (2016). Distal Limb Patterning Requires Modulation of cis-Regulatory Activities by HOX13. Cell reports 17, 2913-2926.
Shibata, A., Machida, J., Yamaguchi, S., Kimura, M., Tatematsu, T., Miyachi, H., Matsushita, M., Kitoh, H., Ishiguro, N., Nakayama, A., et al. (2016). Characterisation of novel RUNX2 mutation with alanine tract expansion from Japanese cleidocranial dysplasia patient. Mutagenesis 31, 61-67.
Shin, Y., Berry, J., Pannucci, N., Haataja, M.P., Toettcher, J.E., and Brangwynne, C.P. (2017). Spatiotemporal Control of Intracellular Phase Transitions Using Light-Activated optoDroplets. Cell 168, 159-171 e114.
Shin, Y., and Brangwynne, C.P. (2017). Liquid phase condensation in cell physiology and disease. Science 357*.*
Staby, L., O'Shea, C., Willemoes, M., Theisen, F., Kragelund, B.B., and Skriver, K. (2017). Eukaryotic transcription factors: paradigms of protein intrinsic disorder. Biochem J 474, 2509-2532.
Stampfel, G., Kazmar, T., Frank, O., Wienerroither, S., Reiter, F., and Stark, A. (2015). Transcriptional regulators form diverse groups with context-dependent regulatory functions. Nature 528, 147-151.
Studier, F.W. (2005). Protein production by auto-induction in high density shaking cultures. Protein Expr Purif 41, 207-234.
Truant, R., Atwal, R.S., Desmond, C., Munsie, L., and Tran, T. (2008). Huntington's disease: revisiting the aggregation hypothesis in polyglutamine neurodegenerative diseases. FEBS J 275, 4252-4262.
Villavicencio-Lorini, P., Kuss, P., Friedrich, J., Haupt, J., Farooq, M., Turkmen, S., Duboule, D., Hecht, J.,and Mundlos, S. (2010). Homeobox genes d11-d13 and a13 control mouse autopod cortical bone and joint formation. The Journal of clinical investigation 120, 1994-2004.
Wang, J., Choi, J.M., Holehouse, A.S., Lee, H.O., Zhang, X., Jahnel, M., Maharana, S., Lemaitre, R., Pozniakovsky, A., Drechsel, D., et al. (2018). A Molecular Grammar Governing the Driving Forces for Phase Separation of Prion-like RNA Binding Proteins. Cell 174, 688-699 e616.
Wheeler, J.R., Lee, H.O., Poser, I., Pal, A., Doeleman, T., Kishigami, S., Kour, S., Anderson, E.N., Marrone, L., Murthy, A.C., et al. (2019). Small molecules for modulating protein driven liquid-liquid phase separation in treating neurodegenerative disease. BioRxiv, http://dx.doi.org/10.1101/721001.
Yu, G., Wang, L.G., Han, Y., and He, Q.Y. (2012). clusterProfiler: an R package for comparing biological themes among gene clusters. OMICS 16, 284-287.
Zamudio, A.V., Dall'Agnese, A., Henninger, J.E., Manteiga, J.C., Afeyan, L.K., Hannett, N.M., Coffey, E.L., Li, C.H., Oksuz, O., Sabari, B.R., et al. (2019). Mediator Condensates Localize Signaling Factors to Key Cell Identity Genes. Molecular cell.
Zoghbi, H.Y., and Orr, H.T. (2000). Glutamine repeats and neurodegeneration. Annu Rev Neurosci 23, 217-247.

## Claims

1. An in vitro method of determining the capacity of a Cluster 1 mammalian Transcription Factor for phase separation and/or for forming a transcriptional condensate.

2. The method of claim 1 wherein the Transcription Factor is a Cluster 1 human Transcription Factor, particularly selected from the group of human Cluster 1 Transcription Factors shown in Table 1 and more particularly selected from the group consisting of HOXD13, HOXA13, RUNX2, SOX3, FOXL2, ZIC2, ARX, PHOX2B, and AR.

3. The method of claim 1 or 2 wherein an altered capacity for phase separation and/or for forming a transcriptional condensate versus the wild-type is indicative for the presence of a disease-associated mutation in the Cluster 1 Transcription Factor.

4. The method of claim 3 wherein the disease-associated mutation comprises an expansion of an amino acid repeat in an intrinsically disordered region (IDR) of the Transcription Factor.

5. The method of claim 4 wherein the expansion comprises an expansion of at least 2, 3, 4, 5, 6, 7, 8, 9, 10, 11, 12, 13, 14, 15 or more amino acid residues, particularly hydrophobic amino acid residues, including but not limited to alanine, in the IDR of the Transcription Factor.

6. The method of any one of claims 1-5 wherein the sample is selected from a body fluid sample, a tissue sample and a cell culture sample.

7. The method of any one of claims 1-6 comprising determining the presence, localization and/or morphology of a transcriptional condensate comprising said Transcription Factor, particularly by an optical method, including but not limited to high-resolution imaging, confocal imaging, and stochastic optical reconstruction microscopy.

8. The method of any one of claims 1-7 comprising determining the composition of a transcriptional condensate comprising said Transcription Factor, particularly by determining the presence and/or amount of at least one transcriptional co-factor such as Mediator or a sub-unit thereof, BRD4 ...

9. The method of any one of claim 1-8 for detecting a genetic disease in a subject wherein an altered capacity for phase separation and/or for forming a transcriptional condensate versus the wild-type is indicative for a genetic disease.

10. The method of any one of claim 1-8 for screening a test compound whether it shows an effect on the capacity for phase separation and/or for forming a transcriptional condensate.

11. The method of claim 10 comprising determining the effect of the test compound on the capacity for phase separation and/or for forming a transcriptional condensate in the presence of a Transcription Factor comprising an altered capacity versus the wild-type.

12. The method of claim 10 or 11 wherein said test compound is an amphiphilic compound comprising
(i) a hydrophobic component comprising a hetero-aromatic nitrogen-containing base, e.g. a purine or pyrimidine base, and
(ii) a hydrophilic component, e.g. comprising a sugar and/or a phosphate group,
and wherein said test compound is particularly a nucleoside phosphate, more particularly a nucleoside triphosphate such as ATP.

13. An amphiphilic compound comprising
(i) a hydrophobic component comprising a hetero-aromatic nitrogen-containing base, e.g. a purine or pyrimidine base, and
(ii) a hydrophilic component, e.g. comprising a sugar and/or a phosphate group,
and wherein said amphiphilic compound is particularly a nucleoside phosphate, more particularly a nucleoside triphosphate such as ATP,
for use in a method of preventing and/or treating a disorder associated with, caused by and/or accompanied with a dysfunction of a transcriptional condensate comprising at least one Cluster 1 mammalian Transcription Factor.

14. An amphiphilic compound for the use of claim 13, wherein the disorder is a genetic disorder, particularly a genetic disorder, more particularly a genetic disorder, which is associated with, caused by and/or accompanied a disease-associated mutation in the Cluster 1 Transcription Factor.

15. In vitro use of an amphiphilic molecule for modulating the capacity of a Cluster 1 mammalian Transcription Factor for phase separation and/or for forming a transcriptional condensate.
